# EUROPÄISCHE PATENTSCHRIFT

(11) **EP 1 888 707 B1**
(45) Veröffentlichungstag und Bekanntmachung des Hinweises auf die Patenterteilung: **09.08.2017**
(21) Anmeldenummer: 06753643.3
(22) Anmeldetag: 16.05.2006
(51) Int. Cl.: C09K 11/06, C07C 15/28, C07D 487/08, H05B 33/14, C07C 15/58, C07D 407/10, H05B 33/18, C07C 15/60, C07D 241/38, H05B 33/20, C07C 23/18, C07C 13/62, H01L 51/00, C07D 493/08, C07D 403/10

(54) **NEUE MATERIALIEN FÜR ORGANISCHE ELEKTROLUMINESZENZVORRICHTUNGEN**
NEW MATERIALS FOR ORGANIC ELECTROLUMINESCENCE DEVICES
NOUVEAUX MATERIAUX DESTINES A DES DISPOSITIFS ELECTROLUMINESCENTS ORGANIQUES

(30) Priorität: 09.06.2005 DE 102005026651
(43) Veröffentlichungstag der Anmeldung: 20.02.2008
(73) Patentinhaber: Merck Patent GmbH, 64293 Darmstadt (DE)
(72) Erfinder: HEIL, Holger, 64285 Darmstadt (DE); BUESING, Arne, 65129 Frankfurt/Main (DE); STOESSEL, Philipp, 60487 Frankfurt am Main (DE); VESTWEBER, Horst, 34630 Gilersberg-Winterscheid (DE)
(86) Internationale Anmeldenummer: PCT/EP2006/004609
(87) Internationale Veröffentlichungsnummer: WO 2006/131192

(56) Entgegenhaltungen:
- EP-A- 1 182 183
- PATENT ABSTRACTS OF JAPAN Bd. 2000, Nr. 05, 14. September 2000 (2000-09-14) -& JP 2000 053676 A (IDEMITSU KOSAN CO LTD), 22. Februar 2000 (2000-02-22)
- PATENT ABSTRACTS OF JAPAN Bd. 2003, Nr. 12, 5. Dezember 2003 (2003-12-05) -& JP 2005 314239 A (MITSUI CHEMICALS INC), 10. November 2005 (2005-11-10)
- PATENT ABSTRACTS OF JAPAN Bd. 2003, Nr. 12, 5. Dezember 2003 (2003-12-05) & JP 2005 008600 A (SONY CORP), 13. Januar 2005 (2005-01-13) in der Anmeldung erwähnt
- ISRAEL AGRANAT, SHMUEL COHEN, ROLAND ISAKSSON, JAN SANDSTROEM, M. RACHEL SUISSA: JOURNAL OF ORGANIC CHEMISTRY, Bd. 55, 1990, Seiten 4943-4950, XP009071673

## Beschreibung

Organische Halbleiter werden als funktionelle Materialien in einer Reihe verschiedenartiger Anwendungen verwendet, die im weitesten Sinne der Elektronikindustrie zugerechnet werden können. Der allgemeine Aufbau organischer Elektrolumineszenzvorrichtungen (OLEDs) ist beispielsweise US 4539507, US 5151629, EP 0676461 und WO 98/27136 beschrieben.

Allerdings zeigen diese Vorrichtungen immer noch erhebliche Probleme, die einer dringenden Verbesserung bedürfen: So ist die operative Lebensdauer insbesondere bei blauer Emission immer noch gering, so dass bis dato nur einfache Anwendungen kommerziell realisiert werden konnten. Weiterhin sind die für blaue Emitter verwendeten Host-Materialien gemäß dem Stand der Technik, die häufig kondensierte aromatische Systeme enthalten, oft nur schwer in gängigen organischen Lösemitteln löslich, was ihre Reinigung bei der Synthese, aber auch die Reinigung der Anlagen bei der Herstellung der organischen elektronischen Vorrichtungen erschwert.

Als nächstliegender Stand der Technik kann die Verwendung verschiedener kondensierter Aromaten, insbesondere Anthracen- oder Pyrenderivate, als Host-Materialien vor allem für blau emittierende Elektrolumineszenzvorrichtungen genannt werden. Als Host-Material gemäß dem Stand der Technik ist 9,10-Bis(2-naphthyl)anthracen (US 5935721) bekannt. Weitere Anthracen-Derivate, die sich als Host-Materialien eignen, sind beispielsweise in WO 01/076323, in WO 01/021729, in WO 04/013073, in WO 04/018588, in WO 03/087023 oder in WO 04/018587 beschrieben. Host-Materialien, basierend auf arylsubstituierten Pyrenen und Chrysenen, werden in WO 04/016575 beschrieben, wobei hier prinzipiell auch entsprechende Anthracen- und Phenanthren-Derivate mit umfasst sind. In WO 03/095445 und in CN 1362464 werden 9,10-Bis(1-naphthyl)anthracen-Derivate für die Verwendung in OLEDs beschrieben. Für hochwertige Anwendungen ist es jedoch notwendig, verbesserte Host-Materialien zur Verfügung zu haben. Insbesondere problematisch ist die schlechte Löslichkeit vieler der genannten Systeme gemäß dem Stand der Technik, was die Herstellung, Reinigung und Verarbeitung der Verbindungen erschwert.

Der oben aufgeführte Stand der Technik belegt, dass das Host-Material eine entscheidende Rolle bei der Funktion organischer Elektrolumineszenzvorrichtungen spielt. Es besteht also weiterhin ein Bedarf an verbesserten Materialien, insbesondere Host-Materialien für blau emittierende OLEDs, die in organischen elektronischen Vorrichtungen zu guten Effizienzen und gleichzeitig zu hohen Lebensdauern führen und die eine gute Löslichkeit aufweisen. Überraschend wurde gefunden, dass organische elektronische Vorrichtungen, die bestimmte kondensierte Aromaten enthalten, die mit Arylgruppen mit kondensierten Cycloalkylgruppen substituiert sind, deutliche Verbesserungen gegenüber dem Stand der Technik aufweisen. Mit diesen Materialien ist eine Steigerung der Lebensdauer der organischen elektronischen Vorrichtung im Vergleich zu Materialien gemäß dem Stand der Technik möglich. Im Gegensatz zu den üblicherweise verwendeten rein aromatischen Verbindungen oder solchen, die höchstens mit kurzen offenkettigen Alkylgruppen, beispielsweise Methylgruppen, substituiert sind, weisen die erfindungsgemäßen Verbindungen eine hohe Löslichkeit in den üblicherweise verwendeten organischen Lösemitteln auf. Im Gegensatz zu Verbindungen, die mit offenkettigen langen Alkylgruppen substituiert sind, lassen sich die erfindungsgemäßen Verbindungen auch problemlos sublimieren. Diese Materialien und deren Verwendung in organischen elektronischen Vorrichtungen sind daher der Gegenstand der vorliegenden Erfindung.
In JP 2005/008600 sind 9,10-Bis(5,6,7,8-tetrahydro-2-naphthyl)anthracen-Derivate als Host oder als Lochtransportverbindung in organischen elektronischen Vorrichtungen beschrieben. Diese Verbindungen sind jedoch nicht für die Herstellung tiefblau emittierender Vorrichtungen geeignet.

Gegenstand der Erfindung sind Verbindungen gemäss dem Anspruch 1. Bevorzugt weist die Verbindung gemäß Formel (1) eine Glasübergangstemperatur T_{g} von größer als 70 °C auf, besonders bevorzugt größer als 100 °C, ganz besonders bevorzugt größer als 130 °C.
Unter der ortho-Position im Sinne dieser Erfindung wird die 1,2-Position an Benzol oder anderen Aromaten verstanden, also Positionen an zwei direkt benachbarten C-Atomen am Aromaten.
Eine Arylgruppe im Sinne dieser Erfindung enthält 6 bis 30 C-Atome; eine Heteroarylgruppe im Sinne dieser Erfindung enthält 2 bis 30 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe im Sinne der unten folgenden Definition verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe im Sinne der folgenden Definition sein.

Unter einer kondensierten Arylgruppe im Sinne dieser Erfindung wird ein Ringsystem mit 10 bis 40 aromatischen Ringatomen verstanden, in dem mindestens zwei aromatische Ringe miteinander "verschmolzen", d. h. durch Anellierung einander ankondensiert sind, also mindestens eine gemeinsame Kante und ein gemeinsames aromatisches π-Elektronensystem aufweisen. Unter einer kondensierten Heteroarylgruppe im Sinne dieser Erfindung wird ein Ringsystem mit 8 bis 40 aromatischen Ringatomen verstanden, in dem mindestens zwei aromatische oder heteroaromatische Ringe, von denen mindestens einer heteroaromatisch ist, einander ankondensiert sind. Diese Ringsysteme können durch R substituiert oder unsubstituiert sein. Beispiele für kondensierte aromatische oder heteroaromatische Ringsysteme sind Naphthalin, Chinolin, Benzothiophen, Anthracen, Phenanthren, Phenanthrolin, Pyren, Perylen, Chrysen, Acridin, etc., während beispielsweise Biphenyl keine kondensierte Arylgruppe darstellt, da dort keine gemeinsame Kante zwischen den beiden Ringsystemen vorliegt. Auch Fluoren stellt beispielsweise kein kondensiertes aromatisches Ringsystem dar, da die beiden Phenyl-Einheiten dort kein gemeinsames aromatisches Ringsystem ausbilden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 30 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die kondensierten Aryl- oder Heteroarylgruppen Ar¹ sind ausgewählt aus der Gruppe bestehend aus Anthracen, Phenanthren, Pyren, Naphthacen, Chrysen, Pentacen, und Perylen, die gegebenenfalls durch R substituiert sein können. Die Substitution mit R kann sinnvoll sein, um besser lösliche Verbindungen zu erhalten oder um die elektronischen Eigenschaften einzustellen. Besonders bevorzugt sind die kondensierten Aryl- oder Heteroarylgruppen Ar¹ ausgewählt aus der Gruppe bestehend aus Anthracen, Phenanthren, Pyren oder Perylen, insbesondere Anthracen oder Pyren, die gegebenenfalls durch R substituiert sein können. Dabei erfolgt die Verknüpfung der Einheiten X und Y am Anthracen bevorzugt über die 2,6-Position oder über die 9,10-Position, besonders bevorzugt über die 9,10-Position. Die Verknüpfung am Pyren erfolgt bevorzugt über die 1,6-, die 1,8-, die 1,3- oder die 2,7-Position, besonders bevorzugt über die 1,6- oder über die 2,7-Position. Die Verknüpfung am Phenanthren erfolgt bevorzugt über die 2,7-, die 3,6-, die 2,9- oder die 2,10-Position, besonders bevorzugt über die 2,7- oder über die 3,6-Position. Die Verknüpfung am Perylen erfolgt bevorzugt über die 3,9-, die 3,10-, die 3,8- oder die 2,8-Position, besonders bevorzugt über die 3,9- oder über die 3,10-Position. Die Verknüpfung am Phenanthrolin erfolgt bevorzugt über die 2,9- oder über die 3,8-Position.

Besonders bevorzugt sind die folgenden Strukturen gemäß Formel (7) bis (12), 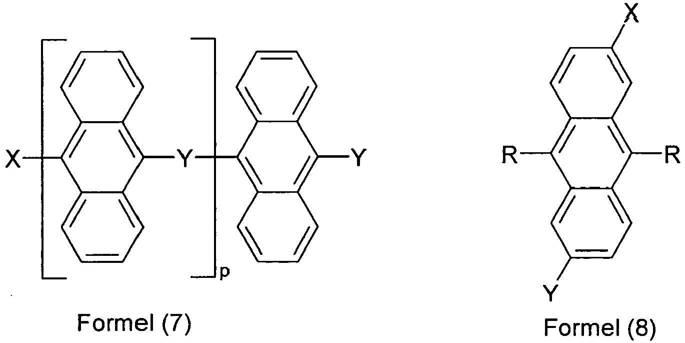 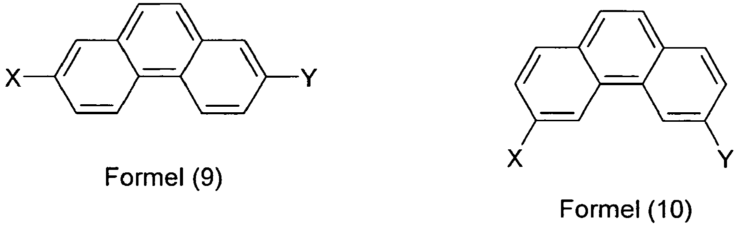 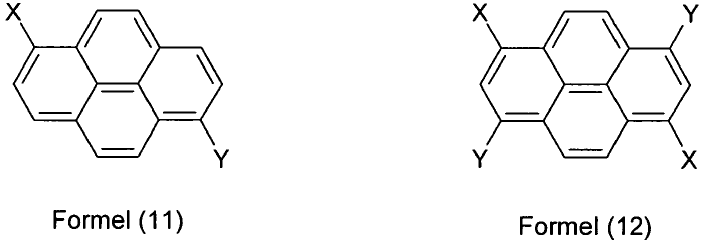 wobei X und Y dieselbe Bedeutung haben, wie oben beschrieben, und wobei die Anthracen- bzw. Phenanthren- bzw. Pyreneinheiten durch einen oder mehrere Reste R substituiert sein können.

Wenn Y eine Gruppe Ar³ darstellt, sind bevorzugte Gruppen Ar³, gleich oder verschieden bei jedem Auftreten, aromatische oder heteroaromatische Ringsysteme mit 5 bis 20 aromatischen Ringatomen, besonders bevorzugt mit 5 bis 16 aromatischen Ringatomen, ganz besonders bevorzugt mit 6 bis 14 aromatischen Ringatomen. Dabei können die Gruppen Ar³ jeweils durch R substituiert oder unsubstituiert sein. Insbesondere bevorzugt sind aromatische Ringsysteme, die keine aromatischen Heteroatome enthalten. Beispiele für besonders bevorzugte Gruppen Ar³ sind Phenyl, 1-Naphthyl, 2-Naphthyl, 2-Phenanthrenyl, 3-Phenanthrenyl, 9-Anthryl, ortho-Biphenyl, meta-Biphenyl oder para-Biphenyl, welche jeweils durch einen oder mehrere Reste R substituiert sein können.

Wenn Y eine Gruppe N(Ar³)₂ darstellt, dann steht Y bevorzugt für eine Gruppe der Formel (13) oder Formel (14) 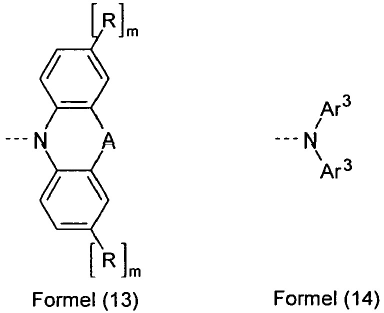 wobei R und m die oben aufgeführte Bedeutung haben und weiterhin gilt:
- A: steht für eine Einfachbindung, O, S, N(R) oder C(R)₂;
- Ar³: ist gleich oder verschieden bei jedem Auftreten eine Aryl- oder Heteroarylgruppe mit 5 bis 20 aromatischen Ringatomen, welche mit einem oder mehreren Resten R substituiert sein kann, bevorzugt eine Aryl- oder Heteroarylgruppe mit 6 bis 14 aromatischen Ringatomen, welche mit einem oder mehreren Resten R substituiert sein kann, besonders bevorzugt Phenyl, 1-Naphthyl oder 2-Naphthyl, welches jeweils mit einem oder mehreren Resten R substituiert sein kann.

Dabei sind die Reste R bevorzugt H, F oder eine Alkylgruppe mit 1 bis 4 C-Atomen.

Strukturen gemäß Formel (2) sind die folgenden Strukturen gemäß den Formeln (15) bis (20), 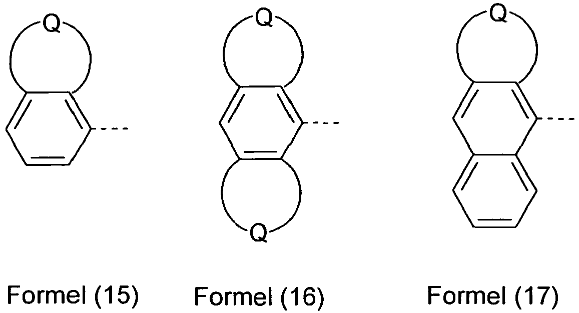 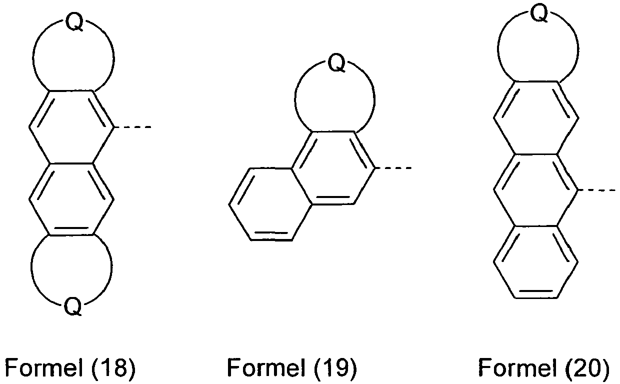 wobei Q dieselbe Bedeutung hat, wie oben beschrieben, und wobei die Phenyl- bzw. Naphthyl- bzw. Anthryleinheit jeweils auch durch R substituiert sein kann; dabei bedeutet die gestrichelte Bindung die Verknüpfung mit der Einheit Ar¹.

In einer bevorzugten Ausführungsform der Erfindung sind die Gruppen X und Y gleich gewählt.

In einer weiteren bevorzugten Ausführungsform der Erfindung sind die Gruppen X und Y unterschiedlich gewählt und Y steht für eine kondensierte Aryl- oder Heteroarylgruppe mit 9 bis 20 aromatischen Ringatomen oder für eine Gruppe N(Ar³)₂.

Bevorzugt ist Q eine lineare Alkylenkette mit 2 bis 15 C-Atomen oder eine verzweigte oder cyclische Alkylengruppe mit 3 bis 15 C-Atomen, die durch R¹ substituiert sein kann und in der auch ein oder mehrere nicht benachbarte C-Atome durch N-R¹, O oder S ersetzt sein können und ein oder mehrere H-Atome durch F oder CN ersetzt sein können. Besonders bevorzugt ist Q eine lineare, verzweigte oder cyclische Alkylenkette mit 3 bis 10 C-Atomen, die durch R¹ substituiert sein kann und in der auch ein oder mehrere nicht benachbarte C-Atome durch N-R¹ oder O ersetzt sein können und ein oder mehrere H-Atome durch F ersetzt sein können.

Ganz besonders bevorzugte Gruppen der Formel (2) sind die im Folgenden abgebildeten Gruppen gemäß den Formeln (21) bis (24), 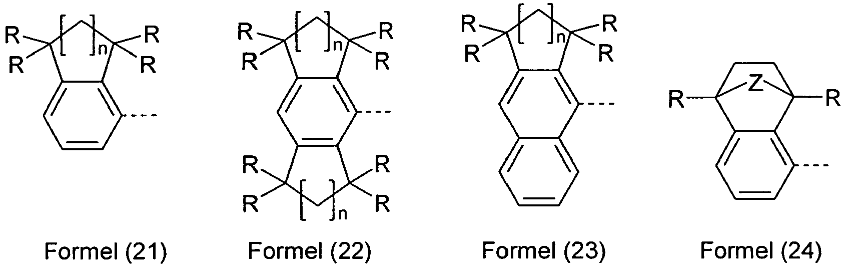 wobei R dieselbe Bedeutung hat, wie oben beschrieben, und weiterhin gilt:
- Z: ist CR₂, O, S, NR, PR, P(=O)R, SiR₂ oder CR₂-CR₂;
- n: ist 1, 2 oder 3, bevorzugt 2;
dabei bedeutet die gestrichelte Bindung die Verknüpfung mit der Einheit Ar¹.

Bevorzugte Strukturen gemäß Formel (21) bis (23) sind solche, in denen der Rest R für eine Gruppe ungleich H oder D steht.
Bevorzugt sind weiterhin Verbindungen gemäß Formel (1), in denen der Index p gleich 0 ist. Manche Verbindungen gemäß Formel (1) können Atropisomere bilden, also Isomere, die durch eine gehinderte Rotation um die X-Ar¹- und um die Ar¹-Y-Bindung zustande kommen. Wenn die Verbindungen gemäß Formel (1) Atropisomere bilden, so umfasst die Erfindung ebenso Mischungen der beiden (oder gegebenenfalls auch mehr) unterschiedlichen Atropisomere wie auch die angereicherten oder reinen Atropisomere der Verbindung.

Beispiele für geeignete Verbindungen gemäß Formel (1) sind die im Folgenden abgebildeten Strukturen (1) bis (98).

| | |
|---|---|
| 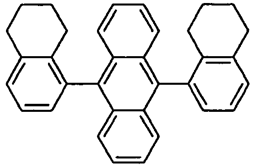 | 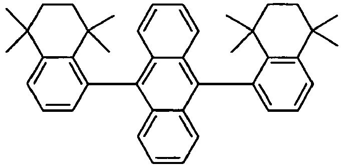 |
| (1) | (2) |
| 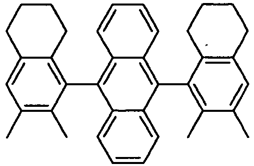 | 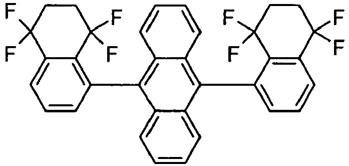 |
| (3) | (4) |
| 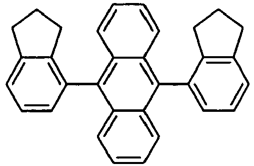 | 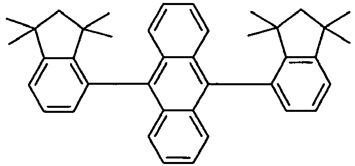 |
| (5) | (6) |
| 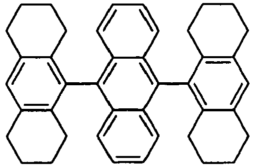 | 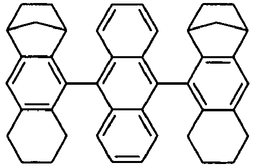 |
| (7) | (8) |
| 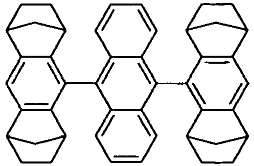 | 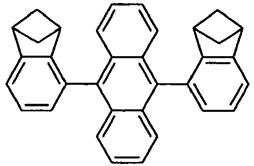 |
| (9) | (10) |
| 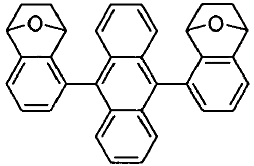 | 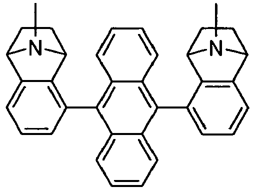 |
| (11) | (12) |
| 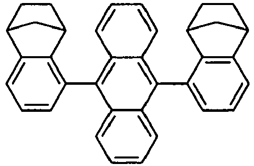 | 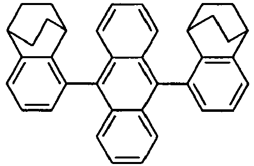 |
| (13) | (14) |
| 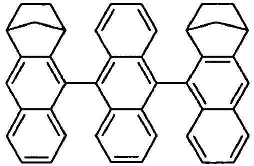 | 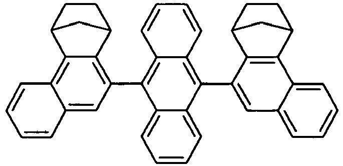 |
| (15) | (16) |
| 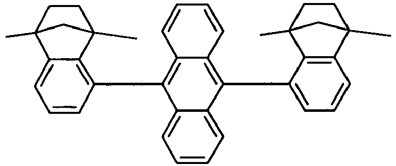 | 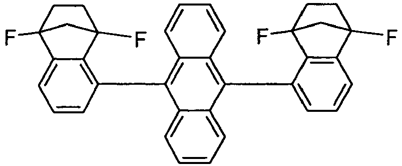 |
| (17) | (18) |
| 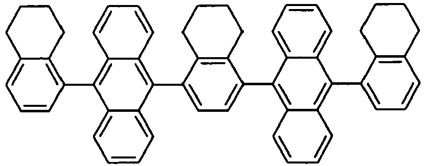 | 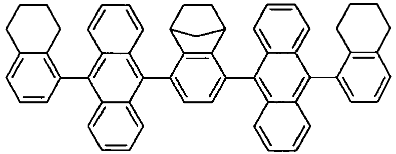 |
| (19) | (20) |
| 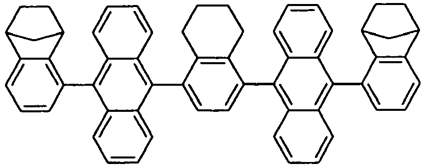 | 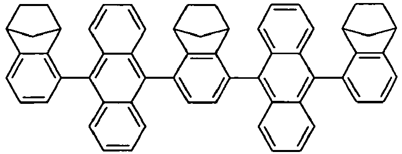 |
| (21) | (22) |
| 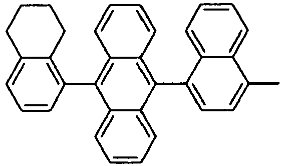 | 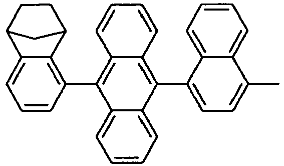 |
| (23) | (24) |
| 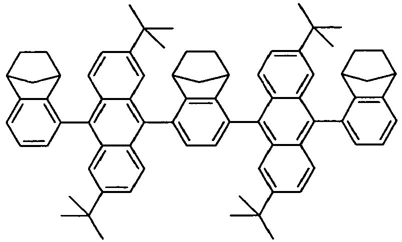 | 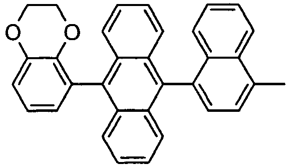 |
| (25) | (26) |
| 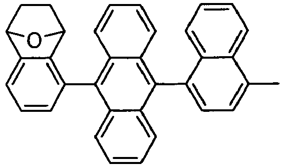 | 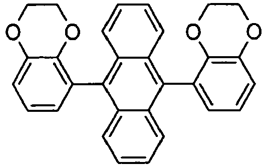 |
| (27) | (28) |
| 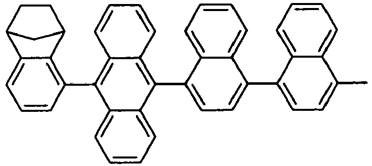 | 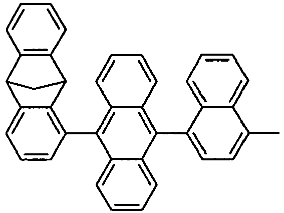 |
| (29) | (30) |
| 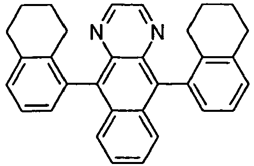 | 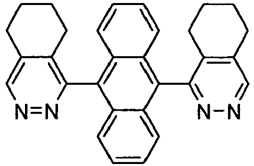 |
| (31) | (32) |
| 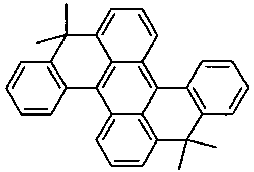 | 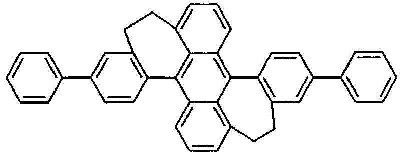 |
| (33) | (34) |
| 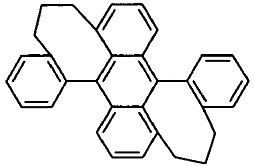 | 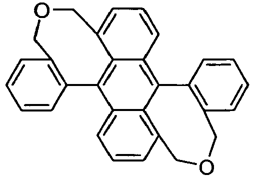 |
| (35) | (36) |
| 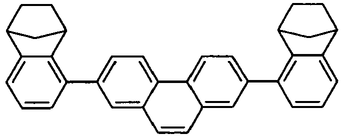 | 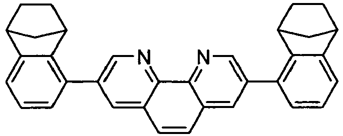 |
| (37) | (38) |
| 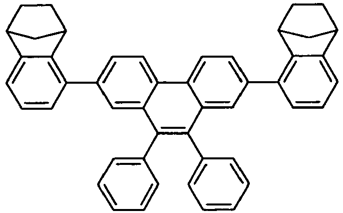 | 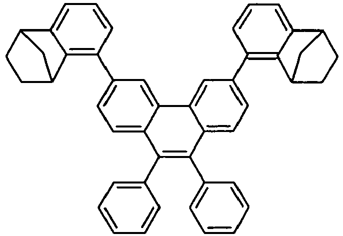 |
| (39) | (40) |
| 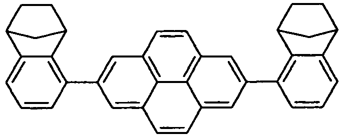 | 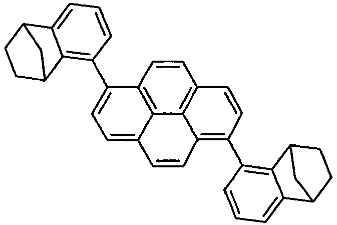 |
| (41) | (42) |
| 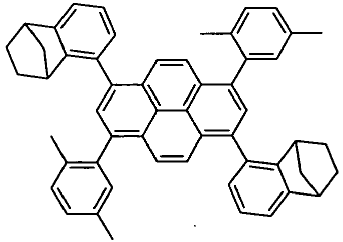 | 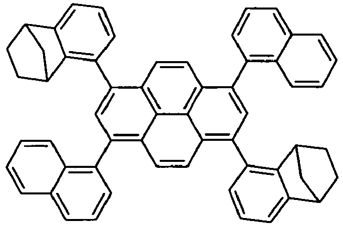 |
| (43) | (44) |
| 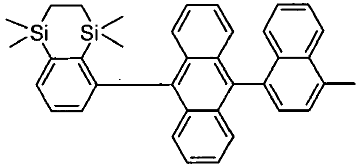 | 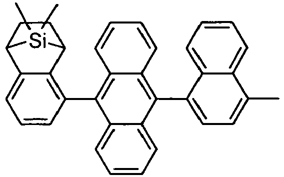 |
| (45) | (46) |
| 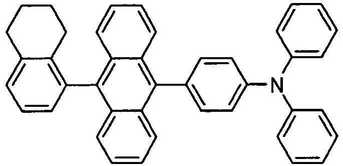 | 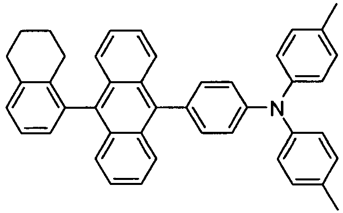 |
| (47) | (48) |
| 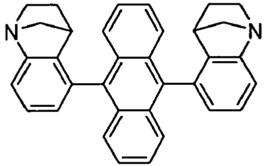 | 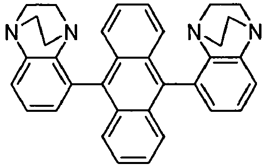 |
| (49) | (50) |
| 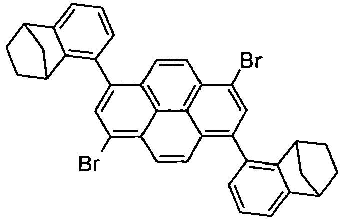 | 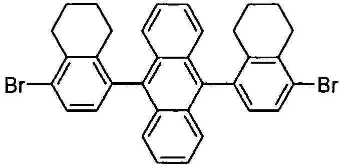 |
| (51) | (52) |
| 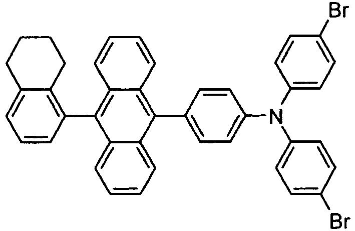 | 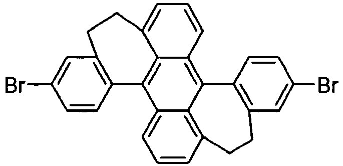 |
| (53) | (54) |
| 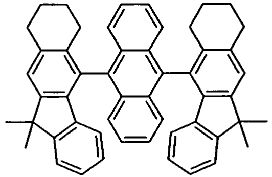 | 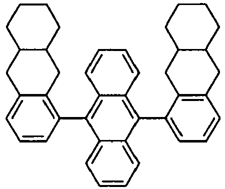 |
| (55) | (56) |
| 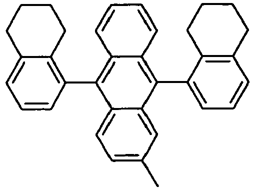 | 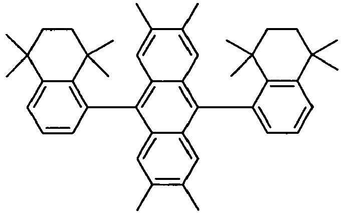 |
| (57) | (58) |
| 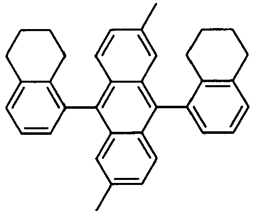 | 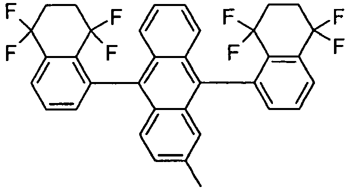 |
| (59) | (60) |
| 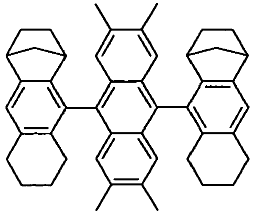 | 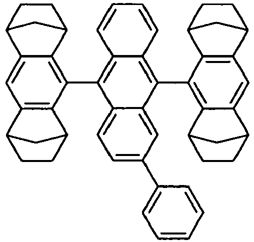 |
| (61) | (62) |
| 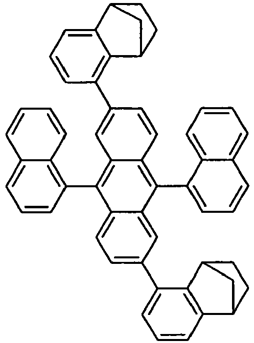 | 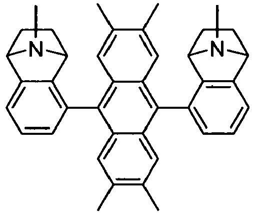 |
| (63) | (64) |
| 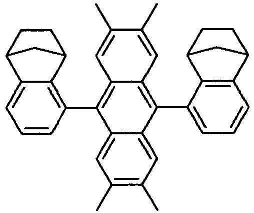 | 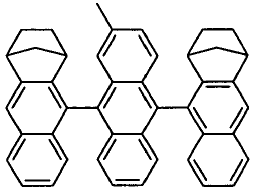 |
| (65) | (66) |
| 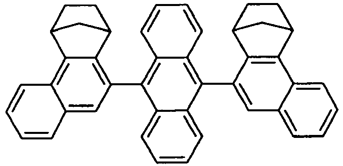 | 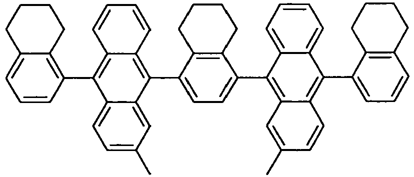 |
| (67) | (68) |
| 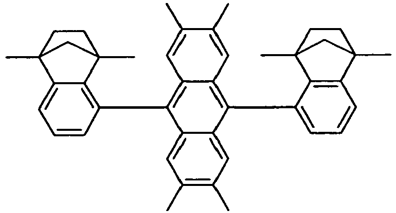 | 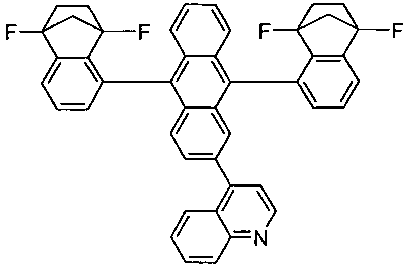 |
| (69) | (70) |
| 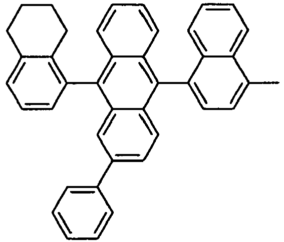 | 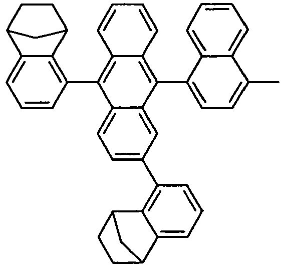 |
| (71) | (72) |
| 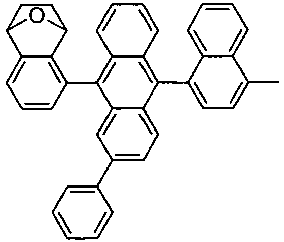 | 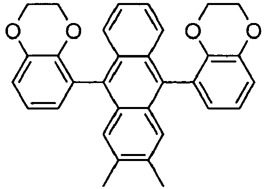 |
| (73) | (74) |
| 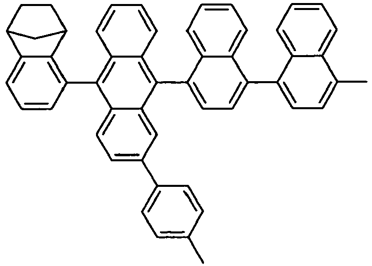 | 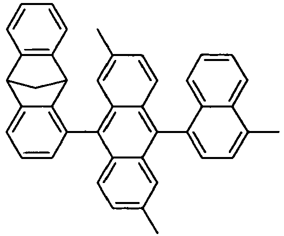 |
| (75) | (76) |
| 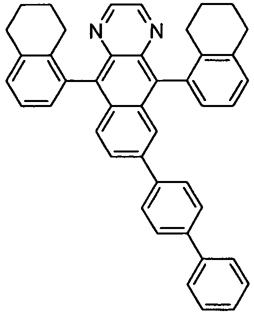 | 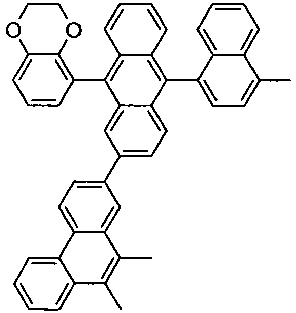 |
| (77) | (78) |
| 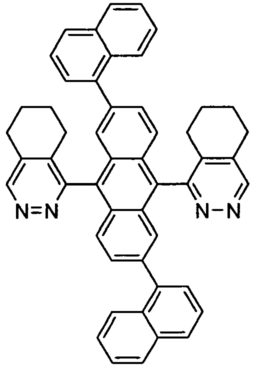 | 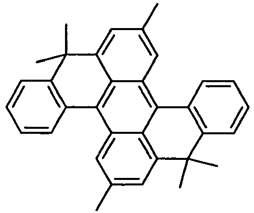 |
| (79) | (80) |
| 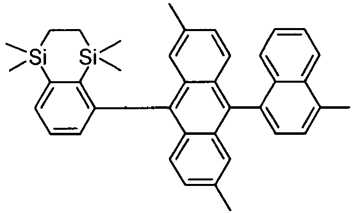 | 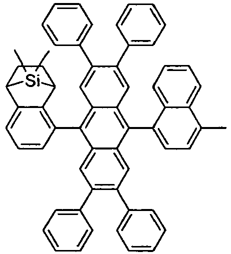 |
| (81) | (82) |
| 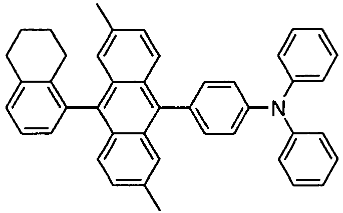 | 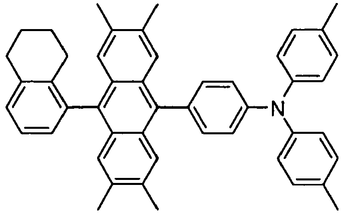 |
| (83) | (84) |
| 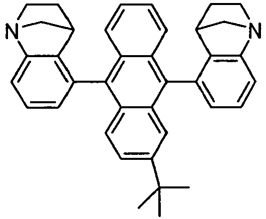 | 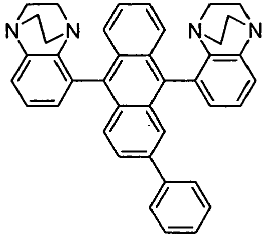 |
| (85) | (86) |
| 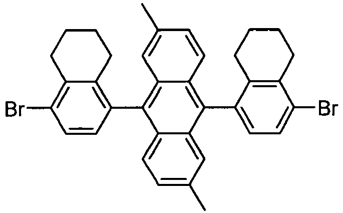 | 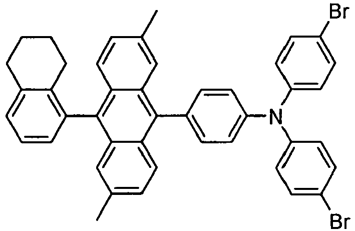 |
| (87) | (88) |
| 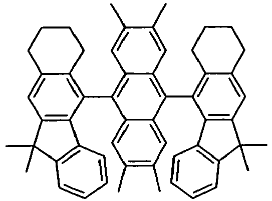 | 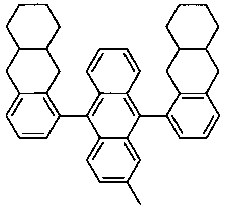 |
| (89) | (90) |
| 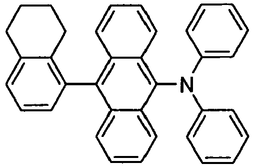 | 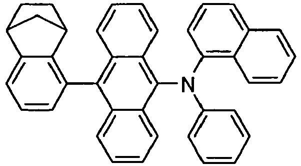 |
| (91) | (92) |
| 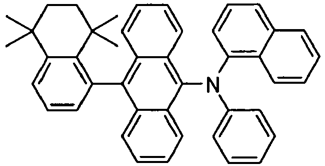 | 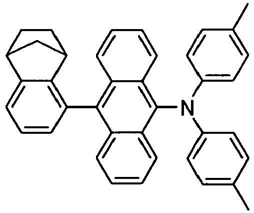 |
| (93) | (94) |
| 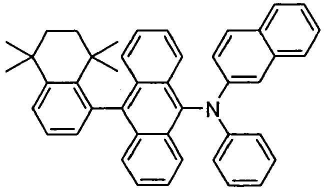 | 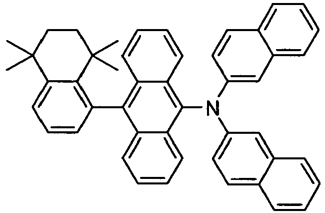 |
| (95) | (96) |
| 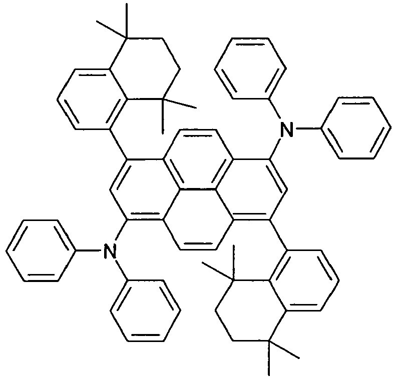 | 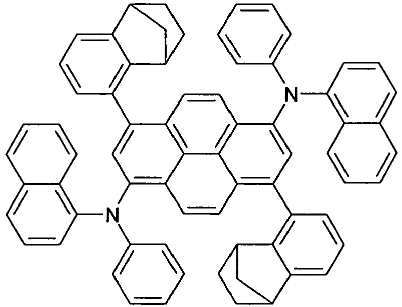 |
| (97) | (98) |

Die Synthese von Verbindungen gemäß Formel (1) kann nach Standardmethoden der organischen Chemie erfolgen. Ein Standardverfahren, das zur Herstellung ähnlicher Systeme gemäß dem Stand der Technik verwendet wird und das auch zur Synthese der erfindungsgemäßen Verbindungen verwendet werden kann, ist die Suzuki-Kupplung zwischen einem aromatischen Halogenid und einem aromatischen Boronsäure-Derivat. So ergibt beispielsweise die Suzuki-Kupplung eines Boronsäure-Derivats der Gruppe X mit einem Dihalogenid eines kondensierten Aromaten Ar¹ symmetrisch substituierte Verbindungen gemäß Formel (1). Unsymmetrisch substituierte Verbindungen gemäß Formel (1) können synthetisiert werden, indem beispielsweise zunächst die Kupplung zwischen X und Ar¹ durchgeführt wird, dann Ar¹ halogeniert wird und mit einem Boronsäure-Derivat von Y gekuppelt wird. Ebenso kann zunächst die Kupplung zwischen Ar¹ und Y durchgeführt werden, dann Ar¹ halogeniert und mit einem Boronsäure-Derivat von X gekuppelt werden. Andere Kupplungsreaktionen sind ebenfalls möglich, beispielsweise Stille-, Negishi-, Sonogashira-, Heck-Kupplung, Grignard-Kreuzkupplung, etc. Die Ausgangsverbindung X in Form des Halogenids lässt sich unter anderem durch direkte Bromierung der entsprechenden Cycloalkylaromaten wie z. B. des 1,2,3,4-Tetrahydronaphthalins zum 5-Brom-1,2,3,4-tetrahydronaphthalin (Ranu et al., Synthetic Communications 1992, 22(8), 1095) oder des Indans zum 4-Brom-2,3-dihydro-1H-indan (Kostermans et al., J. Org. Chem. 1988, 53(19), 4531) darstellen oder auch durch Cycloadditionen aus intermediär erzeugten Arinen wie z. B. im Fall des 5-Brom-1,4-methano-1,2,3,4-tetrahydronaphthalins, das aus 1,3-Dibrom-2-fluorbenzol und Cyclopentadien gewonnen werden kann (Tanida et al., J. Am. Chem. Soc. 1956, 87(21), 4794). Das Arin lässt sich *in situ* nach Methoden herstellen, die dem Fachmann für organische Synthese bekannt sind. Eine weitere Methode zur Herstellung des Arins *in situ* besteht in der Umsetzung eines entsprechenden ortho-Halogenphenols zum entsprechenden Triflat, welches denn mit Magnesium zum Arin umgesetzt und mit einem Dien abgefangen werden kann.

Einige allgemeine Zugangsrouten zu den erfindungsgemäßen Verbindungsklassen sind in den nachfolgenden Schemata dargestellt:

### Darstellung der Substituenten X:

### Tetrahydronaphth-1-yle:

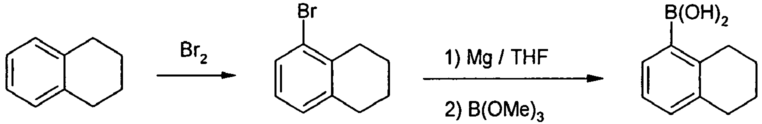

### 1,4-Methano-1,2,3,4-tetrahydronaphth-1-yle

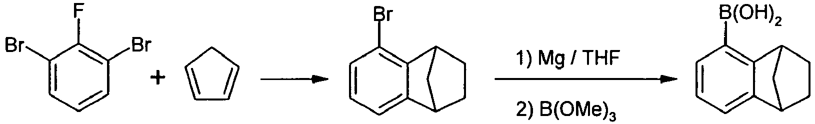

### Kupplung zu den oben abgebildeten Strukturen (1), (13) und (42):

### Struktur (1):

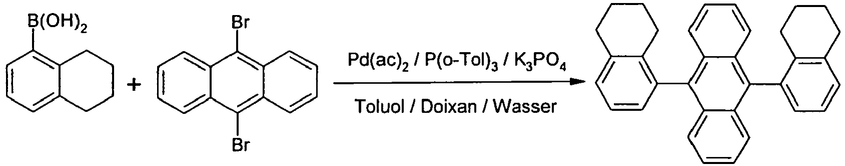

### Struktur (13):

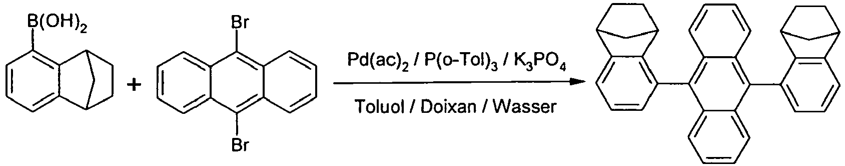

### Struktur (42):

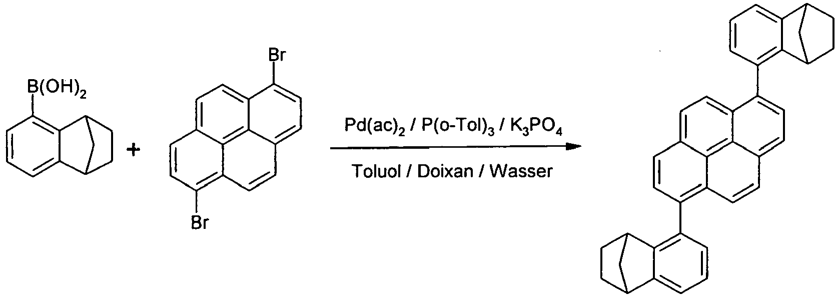

Wenn die Verbindungen gemäß Formel (1) Atropisomere bilden können, kann es sinnvoll sein, die Atropisomere zu separieren, um reine Verbindungen für die Verwendung in organischen elektronischen Vorrichtungen zur Verfügung zu haben. Wie Atropisomere getrennt werden können, ist beispielsweise ausführlich in der nicht offen gelegten Anmeldung EP 04026402.0 beschrieben. Hierfür eignen sich beispielsweise Umkristallisation, Chromatographie oder fraktionierte Sublimation.

Geeignet funktionalisierte Verbindungen gemäß Formel (1), insbesondere bromierte Verbindungen, wie beispielsweise die oben abgebildeten Strukturen (51) bis (56), (87) und (88), können auch zum Einbau in Polymere verwendet werden.

Gegenstand der Erfindung sind daher weiterhin konjugierte, teilkonjugierte oder nicht-konjugierte Polymere, Oligomere oder Dendrimere, enthaltend Wiederholeinheiten gemäß Formel (1). Dabei stellt mindestens ein Rest R an Einheiten gemäß Formel (1) eine Bindung zum Polymer dar. Die Polymere enthalten beispielsweise als weitere Wiederholeinheiten Fluorene (z. B. gemäß EP 842208 oder WO 00/22026), Spirobifluorene (z. B. gemäß EP 707020, EP 894107, WO 03/020790 oder EP 04028865.6), Para-phenylene (z. B. gemäß WO 92/18552), Dihydrophenanthrene (z. B. gemäß WO 05/014689), Phenanthrene (z. B. gemäß WO 05/104264), Indenofluorene (z. B. gemäß WO 04/041901 oder WO 04/113412), Carbazole (z. B. gemäß WO 04/070772 oder WO 04/113468), Anthracene, Naphthaline (z. B. gemäß EP 04030093.1), Triarylamine, Metallkomplexe oder Thiophene (z. B. gemäß EP 1028136) oder auch mehrere dieser Einheiten. Auch Homopolymere der Wiederholeinheiten gemäß Formel (1) sind möglich.

Gegenstand der Erfindung sind weiterhin Mischungen enthaltend mindestens eine Verbindung gemäß Formel (1) und einen oder mehrere Dotanden. Die Dotanden sind bevorzugt ausgewählt aus der Klasse der aromatischen Anthracenamine, der aromatischen Anthracendiamine, der aromatischen Pyrenamine, der aromatischen Pyrendiamine, der Monostyrylamine, der Distyrylamine, der Tristyrylamine, der Tetrastyrylamine, der Styrylphosphine, der Styrylether und der Arylamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine und Pyrendiamine sind analog dazu definiert. Unter einem Monostyrylamin wird eine Verbindung verstanden, die eine Styrylgruppe und mindestens ein, bevorzugt aromatisches, Amin enthält. Unter einem Distyrylamin wird eine Verbindung verstanden, die zwei Styrylgruppen und mindestens ein, bevorzugt aromatisches, Amin enthält. Unter einem Tristyrylamin wird eine Verbindung verstanden, die drei Styrylgruppen und mindestens ein, bevorzugt aromatisches, Amin enthält. Unter einem Tetrastyrylamin wird eine Verbindung verstanden, die vier Styrylgruppen und mindestens ein, bevorzugt aromatisches, Amin enthält. Entsprechende Phosphine und Ether sind in Analogie zu den Aminen definiert. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält. Die Styrylgruppen sind besonders bevorzugt Stilbene, die auch noch weiter substituiert sein können. Bevorzugte Dotanden sind gewählt aus den Klassen der Tristilbenamine, der aromatischen Stilbendiamine, der Anthracendiamine und der Pyrendiamine. Besonders bevorzugte Dotanden sind ausgewählt aus der Klasse der Tristyrylamine und der Stilbendiamine. Beispiele für derartige Dotanden sind substituierte oder unsubstituierte Tristilbenamine oder die Dotanden, die in WO 06/000388 und in den nicht offen gelegten Patentanmeldungen EP 04028407.7 und EP 05001891.0 beschrieben sind.

Gegenstand der Erfindung ist weiterhin die Verwendung von Verbindungen gemäß Formel (1) oder entsprechender Polymere in organischen elektronischen Vorrichtungen.

Nochmals ein weiterer Gegenstand der vorliegenden Erfindung sind organische elektronische Vorrichtungen, enthaltend Anode, Kathode und mindestens eine organische Schicht, welche mindestens eine Verbindung gemäß Formel (1) oder ein entsprechendes Polymer enthält.

Die organische elektronische Vorrichtung ist bevorzugt ausgewählt aus der Gruppe elektronischer Vorrichtungen, bestehend aus organischen und polymeren Leuchtdioden (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren und organischen Laserdioden (O-Laser). Bevorzugt sind organische und polymere Leuchtdioden.

Die organische elektronische Vorrichtung enthält eine oder mehrere organische Schichten, von denen mindestens eine Schicht mindestens eine Verbindung gemäß Formel (1) enthält. Wenn es sich um eine organische Elektrolumineszenzvorrichtung handelt, ist mindestens eine organische Schicht eine Emissionsschicht. Bei organischen Transistoren ist mindestens eine organische Schicht eine Ladungstransportschicht. In organischen Elektrolumineszenzvorrichtungen können außer der emittierenden Schicht noch weitere Schichten vorhanden sein. Diese können beispielsweise sein: Lochinjektionsschicht, Lochtransportschicht, Ladungsblockierschicht, Elektronentransportschicht und/oder Elektroneninjektionsschicht. Es sei aber an dieser Stelle darauf hingewiesen, dass nicht notwendigerweise jede dieser Schichten vorhanden sein muss.

Die Verbindungen gemäß Formel (1) können als Host-Material für Dotanden, die aus dem Singulettzustand oder aus einem Zustand höherer Spinmultiplizität (z. B. dem Triplett-Zustand) Licht emittieren, als Dotand, als Lochtransportmaterial, als Elektronentransportmaterial oder als Lochblockiermaterial verwendet werden. Dabei hängt die bevorzugte Verwendung der Verbindungen gemäß Formel (1) von den vorhandenen Substituenten ab, insbesondere von der Gruppe Y.

Wenn die Gruppe Y für eine Gruppe X oder für ein aromatisches oder heteroaromatisches Ringsystem, insbesondere für eine kondensierte Arylgruppe, steht, wird die Verbindung gemäß Formel (1) bevorzugt als Host-Material zusammen mit einem Dotanden, der aus dem SingulettZustand Licht emittiert, verwendet. Diese Verbindungen eignen sich auch für die Verwendung in einer Elektronentransportschicht und/oder in einer Lochblockierschicht. Bevorzugte Dotanden sind ausgewählt aus der Gruppe der aromatischen Anthracenamine, der aromatischen Anthracendiamine, der aromatischen Pyrenamine, der aromatischen Pyrendiamine, der Monostyrylamine, der Distyrylamine, der Tristyrylamine, der Tetrastyrylamine, der Styrylphosphine, der Styrylether und der Arylamine, wobei diese Verbindungsklassen wie oben definiert sind.

Wenn die Gruppe Y für eine Gruppe N(Ar³)₂ steht, wird die Verbindung gemäß Formel (1) bevorzugt als emittierende Verbindung (emittierender Dotand) eingesetzt. Sie wird dann bevorzugt in Verbindung mit einem Host-Material eingesetzt. Als Hostmaterial eignen sich beispielsweise die oben genannten erfindungsgemäßen Verbindungen, aber auch andere Hostmaterialien, wie sie gemäß dem Stand der Technik üblicherweise verwendet werden. Dies sind insbesondere Oligo-arylene (z. B. 2,2',7,7'-tetraphenyl-spirobifluoren gemäß EP 676461 oder Dinaphthylanthracen), insbesondere Oligo-arylene enthaltend kondensierte aromatische Gruppen, Oligo-arylenvinylene (z. B. DPVBi oder Spiro-DPVBi gemäß EP 676461), polypodale Metallkomplexe (z. B. gemäß WO 04/081017), lochleitende Verbindungen (z. B. gemäß WO 04/058911), elektronenleitende Verbindungen, insbesondere Ketone, Phosphinoxide, Sulfoxide, etc. (z. B. gemäß WO 05/084081 oder WO 05/084082), Atropisomere (z. B. gemäß der nicht offen gelegten Anmeldung EP 04026402.0) oder Boronsäurederivate (z. B. gemäß der nicht offen gelegten Anmeldung EP 05009643.7). Besonders bevorzugte Hostmaterialien sind ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide.

In der Mischung der emittierenden Schicht beträgt der Anteil des Dotanden zwischen 0.1 und 99.0 Gew.%, bevorzugt zwischen 0.5 und 50.0 Gew.%, besonders bevorzugt zwischen 1.0 und 20.0 Gew.%, insbesondere zwischen 1.0 und 10.0 Gew.%. Entsprechend beträgt der Anteil des Hostmaterials in der emittierenden Schicht zwischen 1.0 und 99.9 Gew.%, bevorzugt zwischen 50.0 und 99.5 Gew.%, besonders bevorzugt zwischen 80.0 und 99.0 Gew.%, insbesondere zwischen 90.0 und 99.0 Gew.%.

Wenn die Gruppe Y für eine Gruppe N(Ar³)₂ steht, kann die Verbindung gemäß Formel (1) auch als Lochtransportverbindung eingesetzt werden. Sie wird dann bevorzugt in einer Lochtransportschicht oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht oder auch einer anderen Lochtransportschicht und einer emittierenden Schicht liegt.

Weiterhin bevorzugt ist eine organische elektronische Vorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien in der Regel bei einem Druck zwischen 10⁻⁵ mbar und 1 bar aufgebracht.

Weiterhin bevorzugt ist eine organische elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder InkJet Druck (Tintenstrahl-Druck), hergestellt werden.

Die oben beschriebenen emittierenden Vorrichtungen weisen folgende überraschende Vorteile gegenüber dem Stand der Technik auf:
1. Die Stabilität entsprechender Vorrichtungen ist größer im Vergleich zu Systemen gemäß dem Stand der Technik, was sich vor allem in einer längeren Lebensdauer zeigt.
2. Im Gegensatz zu bisher verwendeten Verbindungen, die durch ihre schlechte Löslichkeit teilweise sehr schwierig zu reinigen waren, sind die Verbindungen gemäß Formel (1) gut löslich und daher einfacher zu reinigen bzw. auch leichter aus Lösung zu verarbeiten.
3. Im Gegensatz zu bisher verwendeten Verbindungen, die keine Substituenten an X in ortho-Position zur Verknüpfung zu Ar¹ aufweisen, lassen sich die erfindungsgemäßen Verbindungen gemäß Formel (1) als Hostmaterialien auch für tiefblaue Emitter einsetzen, während ähnliche Materialien gemäß dem Stand der Technik, wie z. B. gemäß JP 2005/008600, nur für hellblaue Emitter geeignet sind.
4. Die Verwendung der erfindungsgemäßen Verbindungen in OLEDs führt zu einer höheren Effizienz der Lichtemission.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von der Firma ALDRICH bzw. ABCR (Tris(dibenzylidenaceton)dipalladium(0), 2-Dicyclohexylphosphino-2,6-dimethyoxybiphenyl, 9,10-Dibromanthracen, 1,6-Dibrompyren, 1,3,6,8-Tetrabrom-pyren, Anorganika, Lösemittel) bezogen werden. 5-Brom-1,2,3,4-tetrahydro-1,4-methanonaphthalin wird nach Tanida *et al.*, *J. Am. Chem. Soc.* 1965, *87*(*21*), 4794, 5-Brom-1,1,4,4-tetramethyl-1,2,3,4-tetrahydro-8-methylnaphthalin wird in Analogie zu 6-Brom-1,1,4,4-tetramethyl-1,2,3,4-tetrahydronaphthalin nach Garipova *et al.*, *Tetrahedron* **2005**, *61(20)*, 4755 dargestellt. 5-Brom-1,2,3,4-tetrahydronaphthalin wird gemäß Synthetic Communications 1992, 22(8), 1095-1099 synthetisiert. (5,6,7,8-Tetrahydro-1-naphthyl)-boronsäure wird gemäß US 2002/019527 synthetisiert.

### Beispiel 1: 9,10-Bis-(1,2,3,4-tetrahydro-1,4-methano-naphth-5-yl)-anthracen

### a) 1,2,3,4-Tetrahydro-1,4-methano-naphthalin-5-boronsäure

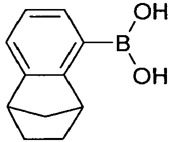

Eine auf -78 °C gekühlte Lösung von 44.6 g (200 mmol) 5-Brom-1,2,3,4-tetrahydro-1,4-methanonaphthalin in 500 ml THF wird tropfenweise mit 100 ml (250 mmol) n-Buthyllithium (2.5 M in.Hexan) versetzt. Die Reaktionsmischung wird 1 h bei -78 °C gerührt und dann schnell mit einer Mischung von 33.5 ml (300 mmol) Trimethylborat in 50 ml THF versetzt. Nach Erwärmen auf -10 °C wird mit 10 ml 2.5 M Salzsäure hydrolysiert, dann mit 500 ml Methyl-tert-butylether versetzt. Die organische Phase wird abgetrennt, mit Wasser gewaschen, über Natriumsulfat getrocknet und zur Trockene eingeengt. Der Rückstand wird in 200 ml n-Heptan aufgenommen, der farblose Feststoff wird abgesaugt, mit n-Heptan gewaschen und im Vakuum getrocknet. Ausbeute: 24.1 g (128 mmol), 64.1 % d. Th.; Reinheit: 98 % n. ¹H-NMR.

### b) 9,10-Bis-(1,2,3,4-tetrahydro-1,4-methano-naphth-5-yl)anthracen

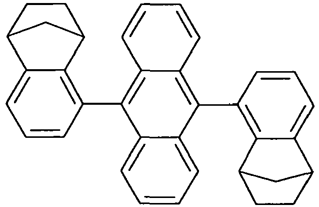

Eine Suspension von 16.8 g (50 mmol) 9,10-Dibromanthracen, 21.6 g (115 mol) 1,2,3,4-Tetrahydro-1,4-methano-naphthalin-5-boronsäure und 66.9 g (315 mmol) Trikaliumphosphat in 400 ml wasserfreiem Toluol wird mit 915 mg (1 mmol) Tris(dibenzylidenaceton)-di-palladium(0) und 821 mg (2 mmol) 2-Dicyclohexylphosphino-2,6-dimethyoxybiphenyl versetzt und 16 h unter Rückfluss erhitzt. Nach Erkalten der Reaktionsmischung werden 400 ml Wasser zugesetzt, der Niederschlag wird abgesaugt, dreimal mit je 200 ml Wasser gewaschen, dreimal mit je 200 ml Ethanol gewaschen, im Vakuum getrocknet und anschließend an Kieselgel chromatographiert (Laufmittel Heptan / Toluol 8:2, v:v, Säulentemperatur 50 °C). Sublimation: p = 1 x 10⁻⁵ mbar, 300 °C. Ausbeute: 13.1 g (28 mmol), 56.6 % d. Th.; Reinheit: 99.5 % n. ¹H-NMR (alle Isomere umfassend).

### Beispiel 2: 1,6-Bis-(1,2,3,4-tetrahydro-1,4-methano-naphth-5-yl)pyren

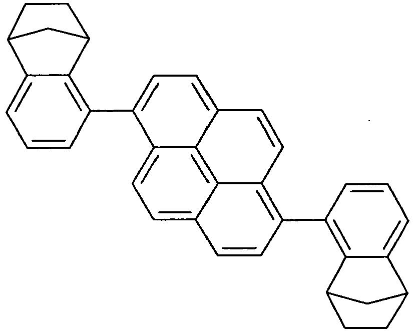

Darstellung analog Beispiel 1. Anstelle von 9,10-Dibromanthracen werden 18.0 g (50 mmol) 1,6-Dibrompyren verwendet. Reinigung durch Umkristallisation aus NMP. Ausbeute: 16.8 g (34.5 mmol), 69.0 % d. Th.; Reinheit: 99.9 % n. ¹H-NMR.

### Beispiel 3: 9,10-Bis-(5,6,7,8-tetrahydro-1-naphthyl)anthracen

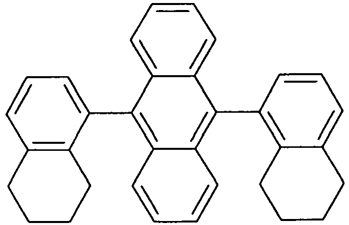

Eine gut gerührte, entgaste Suspension aus 12.7 g (37.7 mmol) 9,10-Dibromanthracen, 17.2 g (97.7 mmol) (5,6,7,8-Tetrahydro-1-naphthyl)-boronsäure und 26.6 g (126 mmol) Trikaliumphosphat in einem Gemisch aus 230 ml Toluol, 115 ml Dioxan und 170 ml Wasser wird mit 688 mg (2.26 mmol) Tri-o-tolylphosphin und dann mit 84 mg (0.37 mmol) Palladium(II)-acetat versetzt und 60 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und im Vakuum zur Trockene eingeengt. Der so erhaltene graue Rückstand wird aus Dioxan umkristallisiert. Die ausgefallenen Kristalle werden abgesaugt, mit 50 ml Ethanol gewaschen und im Vakuum getrocknet; Ausbeute: 7.5 g, 45% mit einer Reinheit von 99.8 % nach HPLC.

Analog zu den Beispielen 1 bis 3 werden folgende Verbindungen dargestellt.

| **Beispiel** | **Arylbromid** | **Produkt** |
|---|---|---|
| 4 | 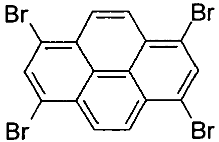 | 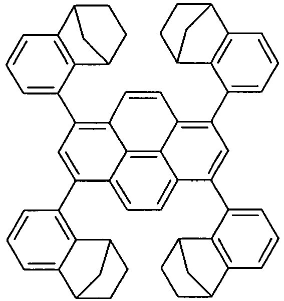 |
| 5 | 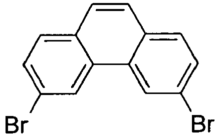 | 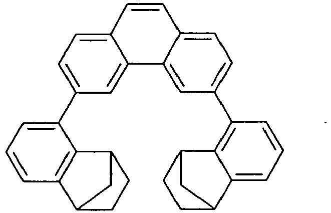 |
| 6 | 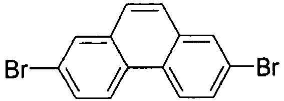 | 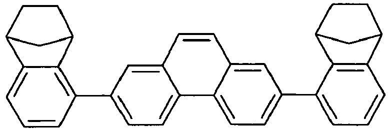 |
| 7 | 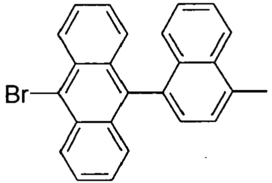 | 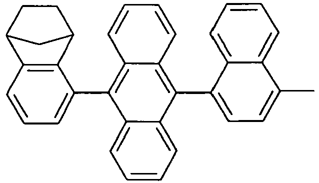 |
| 8 | 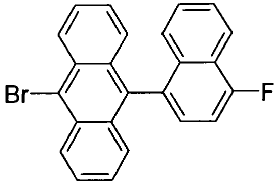 | 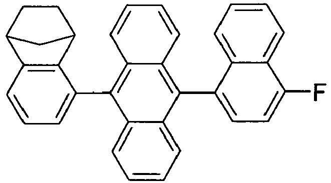 |
| 9 | 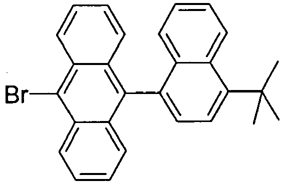 | 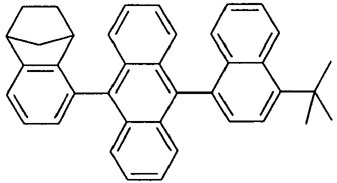 |
| 10 | 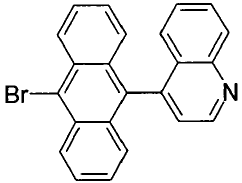 | 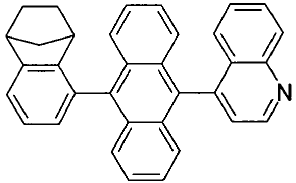 |
| 11 | 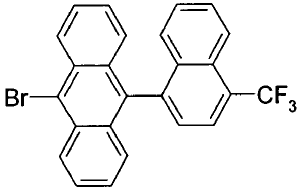 | 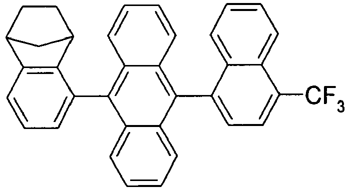 |
| 12 | 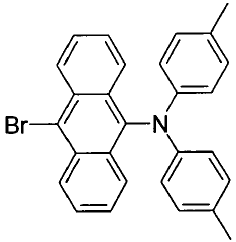 | 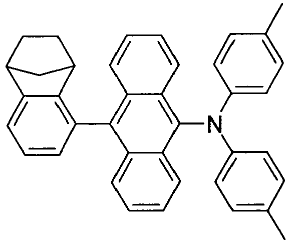 |

### Beispiel 13: 9,10-Bis-(1,1,4,4-tetramethyl-1,2,3,4-tetrahydro-8-methyl-naphth-5-yl)anthracen

### a) 1,1,4,4-Tetramethyl-1,2,3,4-tetrahydro-8-methyl-naphthalin-5-boronsäure

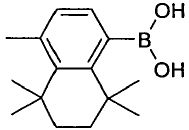

Darstellung analog zu Beispiel 1a. Anstelle von 5-Brom-1,2,3,4-tetrahydro-1,4-methanonaphthalin werden 56.2 g (200 mmol) 5-Brom-1,1,4,4-tetramethyl-1,2,3,4-tetrahydro-8-methyl-naphthalin eingesetzt. Ausbeute: 36.5 g (148 mmol), 74.1 % d. Th.; Reinheit: 98 % n. ¹H-NMR.

### b) 9,10-Bis-(1,1,4,4-tetramethyl-1,2,3,4-tetrahydro-8-methyl-naphthalin-5-yl)anthracen

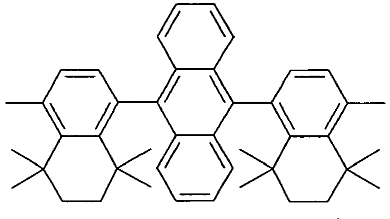

Darstellung analog zu Beispiel 1b. Anstelle von 21.6 g (115 mmol) 1,2,3,4-Tetrahydro-1,4-methanonaphthalin-5-boronsäure werden 36.9 g (150 mmol) 1,1,4,4-Tetramethyl-1,2,3,4-tetrahydro-8-methyl-naphthalin-5-boronsäure verwendet. Sublimation: p = 1 x 10⁻⁵ mbar, 310 °C.Ausbeute: 15.9 g (27.5 mmol), 54.9 % d. Th.; Reinheit: 99.9 % n. ¹H-NMR, atropisomerenrein.

Analog zu Beispiel 13 werden folgende Verbindungen dargestellt:

| **Beispiel** | **Arylbromid** | **Produkt** |
|---|---|---|
| 14 | 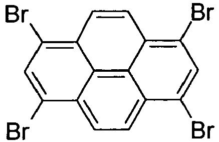 | 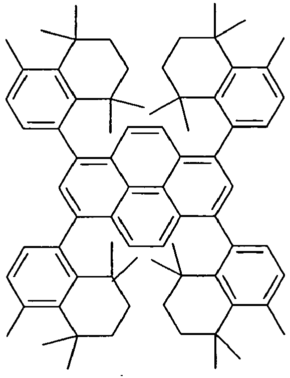 |
| 15 | 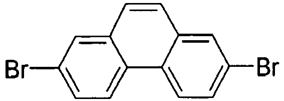 | 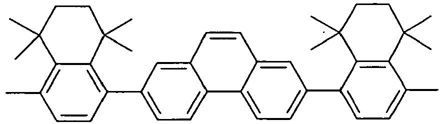 |
| 16 | 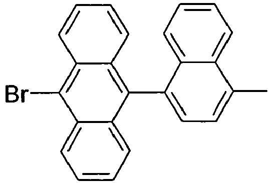 | 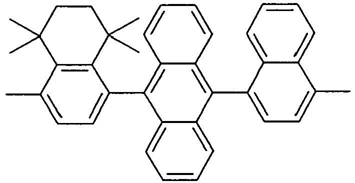 |
| 17 | 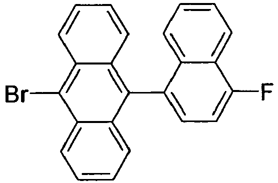 | 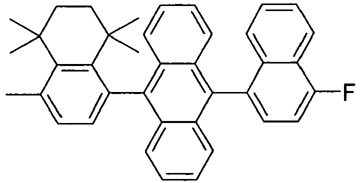 |
| 18 | 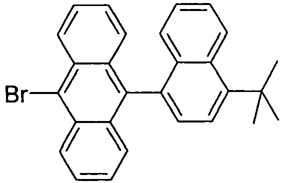 | 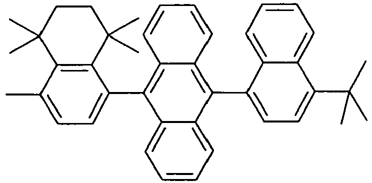 |
| 19 | 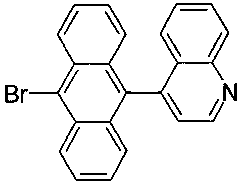 | 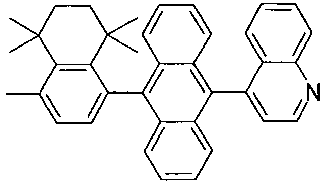 |
| 20 | 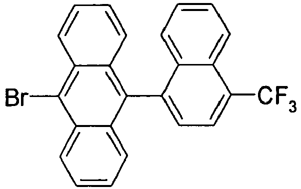 | 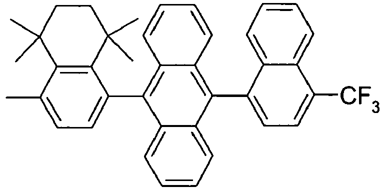 |
| 21 | 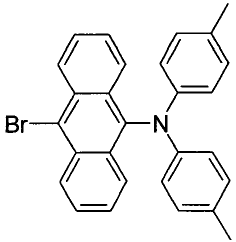 | 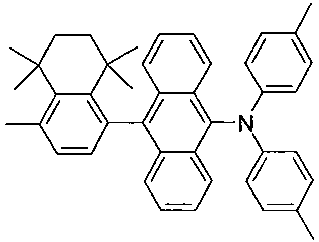 |

### Beispiel 22: Herstellung von fluoreszierenden OLEDs mit erfindungsgemäßen Hostmaterialien H1- H6 für blau elektrolumineszierende OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 23 bis 42 werden die Ergebnisse verschiedener OLEDs vorgestellt. Der grundlegende Aufbau und die verwendeten Materialien (außer der emittierenden Schicht) sind in den Beispielen zur besseren Vergleichbarkeit identisch. Analog dem o. g. allgemeinen Verfahren werden OLEDs mit folgendem Aufbau erzeugt:
- Lochinjektionsschicht (HIL): 20 nm PEDOT (aus Wasser aufgeschleudert; bezogen von H. C. Starck, Goslar, Deutschland; Poly(3,4-ethylendioxy-2,5-thiophen))
- Lochtransportschicht (HTM1): 20 nm 2,2',7,7'-Tetrakis(di-para-tolylamino)spiro-9,9'-bifluoren (aufgedampft);
- Lochtransportschicht (HTM2): 20 nm NPB (N-Naphthyl-N-phenyl-4,4'-diaminobiphenyl)
- Emissionschicht (EML): 30 nm, Schicht aus H1 bis H7 als Hostmaterial dotiert mit x % (s. Tabelle) Dotand E1 (aufgedampft, synthetisiert nach WO 06/000388)
- Elektronenleiter (ETL): 20 nm (aufgedampft; AlQ₃ bezogen von SynTec; Tris(chinolinolato)-aluminium(III))
- Kathode: 1 nm LiF, darauf 150 nm Al.

Die OLEDs können auch ohne PEDOT als Lochinjektionsschicht hergestellt werden. In diesem Fall dient dann das HTM1 als Lochinjektionsschicht. Mit diesen OLEDs werden vergleichbare Ergebnisse erhalten.

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien).

Im Folgenden sind die verwendeten Hostmaterialen (**H1** bis **H7**) und das verwendete Emittermaterial (**E1**) aufgelistet. Dabei dient der Host **H7** als Vergleichsmaterial gemäß dem Stand der Technik.

| | | | |
|---|---|---|---|
| **Emitter E1** | 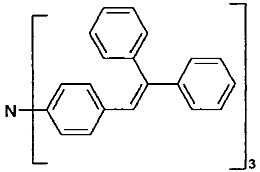 | **Host H1** | 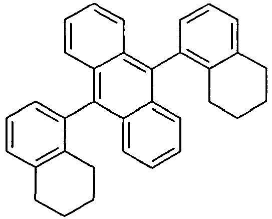 |
| **Host H2** | 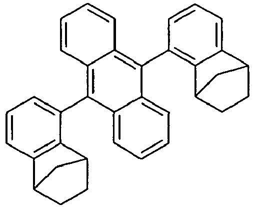 | **Host H3** | 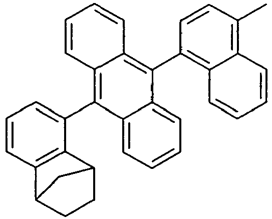 |
| **Host H4** | 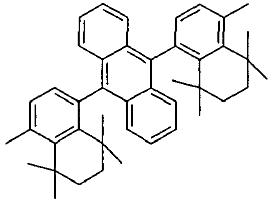 | **Host H5** | 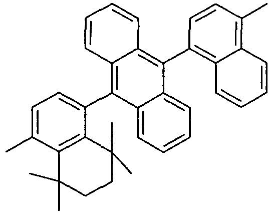 |
| **Host H6** | 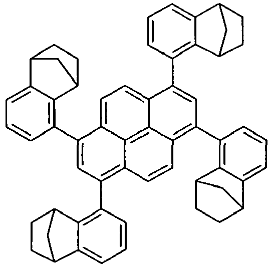 | **Host H7** | 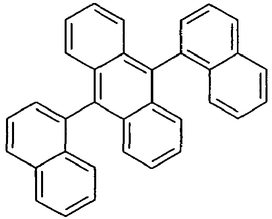 |

In Tabelle 1 sind die Ergebnisse einiger OLEDs (Beispiele 23 bis 42) aufgeführt. Wie man den Beispielen in der Tabelle 1 entnehmen kann, zeigen OLEDs enthaltend den erfindungsgemäßen Hostmaterialen (**H1** bis **H6**) in Kombination mit dem Emitter **E1** eine effiziente blaue Emission. Dabei erhält man eine höhere Effizienz und eine tiefer blaue Farbe als mit Di-1-naphthylanthracen gemäß dem Stand der Technik.

**Tabelle 1**

| **Beispiel** | **EML** | **Max Effizienz (cd/A)** | **Spannung (V) bei 1000cd/m²** | **CIE** |
|---|---|---|---|---|
| 23 | **H1** | 7.7 | 6.3 | x=0.16; |
| | 5 % **E1** | | | y=0.25 |
| 24 | **H1** | 6.9 | 6.5 | x=0.16; |
| | 3 % **E1** | | | y=0.21 |
| 25 | **H2** | 7.2 | 6.2 | x=0.16; |
| | 3 % **E1** | | | y=0.21 |
| 26 | **H2** | 8.1 | 6.1 | x=0.16; |
| | 5 % **E1** | | | y=0.25 |
| 27 | **H2** | 7.8 | 6.0 | x=0.16; |
| | 7 % **E1** | | | y=0.27 |
| 28 | **H3** | 7.7 | 6.2 | x=0.16; |
| | 3 % **E1** | | | y=0.22 |
| 29 | **H3** | 8.2 | 6.0 | x=0.16; |
| | 5 % **E1** | | | y=0.25 |
| 30 | **H3** | 7.5 | 6.3 | x=0.16; |
| | 7 % **E1** | | | y=0.26 |
| 31 | **H4** | 7.1 | 6.4 | x=0.16; |
| | 3 % **E1** | | | y=0.22 |
| 32 | **H4** | 8.1 | 6.0 | x=0.16; |
| | 5 % **E1** | | | y=0.25 |
| 33 | **H4** | 7.2 | 6.2 | x=0.16; |
| | 7 % **E1** | | | y=0.29 |
| 34 | **H5** | 7.5 | 6.2 | x=0.16; |
| | 3 % **E1** | | | y=0.22 |
| 35 | **H5** | 8.3 | 6.1 | x=0.16; |
| | 5 % **E1** | | | y=0.25 |
| 36 | **H5** | 7.5 | 6.1 | x=0.16; |
| | 7 % **E1** | | | y=0.28 |
| 37 | **H6** | 7.7 | 6.6 | x=0.16; |
| | 3 % **E1** | | | y=0.29 |
| 38 | **H6** | 8.5 | 6.5 | x=0.16; |
| | 5 % **E1** | | | y=0.32 |
| 39 | **H6** | 7.0 | 6.1 | x=0.16; |
| | 7 % **E1** | | | y=0.33 |
| 40 | **H7** | 6.3 | 6.9 | x=0.16; |
| (Vergleich) | 3 % **E1** | | | y=0.23 |
| 41 | **H7** | 7.8 | 6.1 | x=0.16; |
| (Vergleich) | 5 % **E1** | | | y=0.28 |
| 42 | **H7** | 6.5 | 6.0 | x=0.16; |
| (Vergleich) | 7 % **E1** | | | y=0.30 |

### Beispiel 43:

Im Folgenden werden Beispiele für OLEDs aufgeführt, die erfindungsgemäße Emitter enthalten. Im Folgenden sind die verwendeten erfindungsgemäßen Emitter **E2** und **E3** aufgelistet:

| | | | |
|---|---|---|---|
| **Emitter 2 E2** | 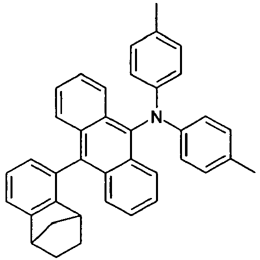 | **Emitter 3 E3** | 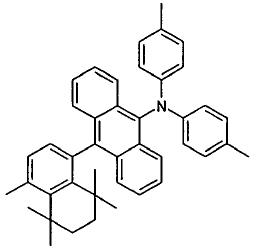 |

In Tabelle 2 sind die Ergebnisse einiger OLEDs (Beispiele 44 bis 50) Wie man den Beispielen in der Tabelle 2 entnehmen kann, zeigen OLEDs enthaltend den erfindungsgemäßen Emitter **E2** oder **E3** gute Effizienzen und gute blaue Farbkoordinaten. Weiterhin weisen die erfindungsgemäßen Emitter **E2** und **E3** eine höhere thermische Stabilität auf als der Emitter **E1** gemäß dem Stand der Technik.

**Tabelle 2**

| **Beispiel** | **EML** | **Max Effizienz (cd/A)** | **Spannung (V) bei 1000cd/m²** | **CIE** |
|---|---|---|---|---|
| 44 | **H7** | 6.5 | 6.5 | x=0.16; |
| | 5 % **E2** | | | y=0.20 |
| 45 | **H7** | 6.6 | 6.4 | x=0.16; |
| | 5 % **E3** | | | y=0.18 |
| 46 | **H7** | 6.8 | 6.3 | x=0.16; |
| | 7 % **E3** | | | y=0.21 |
| 47 | **H2** | 6.8 | 6.4 | x=0.15; |
| | 5 % **E2** | | | y=0.20 |
| 48 | **H2** | 6.9 | 6.5 | x=0.16; |
| | 5 % **E3** | | | y=0.20 |
| 49 | **H5** | 6.7 | 6.4 | x=0.16; |
| | 5 % **E2** | | | y=0.21 |
| 50 | **H5** | 6.8 | 6.3 | x=0.16; |
| | 5 % **E3** | | | y=0.20 |

## Patentansprüche

1. Verbindungen gemäß Formel (1), 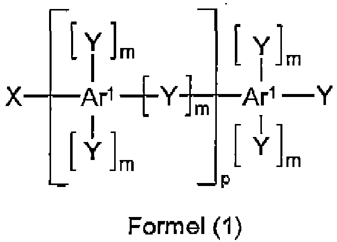 wobei für die verwendeten Symbole und Indizes gilt:
Ar¹ ist bei jedem Auftreten gleich oder verschieden eine kondensierte Aryl- oder Heteroarylgruppe, ausgewählt aus der Gruppe bestehend aus Anthracen, Phenanthren, Pyren, Naphthacen, Chrysen, Pentacen und Perylen, welche durch R substituiert sein können;
X ist bei jedem Auftreten gleich oder verschieden eine Gruppe gemäß einer der Formeln (15) bis (20) 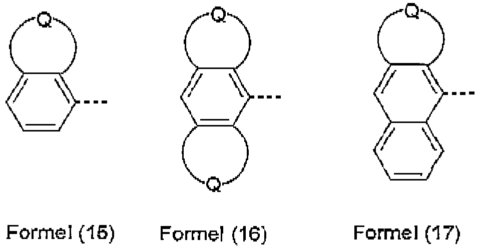 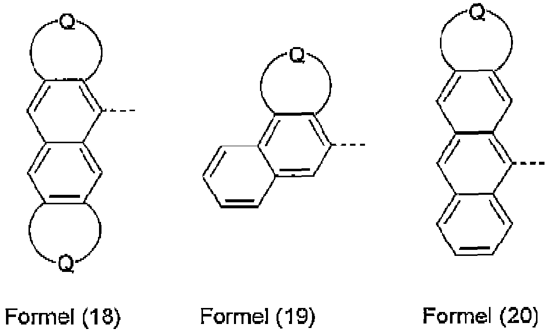 wobei die Phenyl- bzw. Naphthyl- bzw. Anthryleinheit jeweils auch durch R substituiert sein kann; dabei bedeutet die gestrichelte Bindung die Verknüpfung mit der Einheit Ar¹;
Y ist bei jedem Auftreten gleich oder verschieden X, eine Gruppe Ar³ oder eine Gruppe N(Ar³)₂, wobei die beiden Reste Ar³ auch durch eine Einfachbindung oder eine Gruppe O, S, N(R) oder C(R)₂ miteinander verbunden sein können;
Ar³ ist bei jedem Auftreten gleich oder verschieden ein aromatisches oder heteroaromatisches Ringsystem, welches mit einem oder mehreren Resten R substituiert sein kann;
Q ist bei jedem Auftreten gleich oder verschieden eine lineare, verzweigte oder cyclische Alkylen- oder Alkylidengruppe; dabei enthält Q 1 bis 20 C-Atome und kann durch R¹ substituiert sein, und es können auch ein oder mehrere nicht benachbarte C-Atome durch N-R¹, O, S, O-CO-O, CO-O, -CR¹=CR¹- oder -C≡C- ersetzt sein, und ein oder mehrere H-Atome können durch F, Cl, Br, I oder CN ersetzt sein;
R ist bei jedem Auftreten gleich oder verschieden H, F, Cl, Br, I, CN, eine geradkettige Alkyl- oder Alkoxykette mit 1 bis 40 C-Atomen oder eine verzweigte oder cyclische Alkyl- oder Alkoxygruppe mit 3 bis 40 C-Atomen, die jeweils durch R¹ substituiert sein kann, in der auch ein oder mehrere nicht benachbarte C-Atome durch N-R¹, O, S, O-CO-O, CO-O oder -C≡C- ersetzt sein können und in der auch ein oder mehrere H-Atome durch F, Cl, Br, I oder CN ersetzt sein können, oder ein aromatisches oder heteroaromatisches Ringsystem mit 5 bis 40 aromatischen Ringatomen, welches auch durch einen oder mehrere Reste R¹ substituiert sein kann, oder eine Kombination aus zwei, drei oder vier dieser Systeme; dabei können zwei oder mehrere Reste R miteinander auch ein weiteres mono- oder polycyclisches, aliphatisches oder aromatisches Ringsystem bilden;
R¹ ist bei jedem Auftreten gleich oder verschieden H oder ein Kohlenwasserstoffrest mit 1 bis 20 C-Atomen, der aliphatisch oder aromatisch oder eine Kombination aus aliphatisch und aromatisch sein kann und in dem auch ein oder mehrere H-Atome durch F ersetzt sein können;
m ist bei jedem Auftreten 0 oder 1;
p ist bei jedem Auftreten 0 oder 1.

2. Strukturen gemäß Formel (7) bis (12) gemäß Anspruch 1, 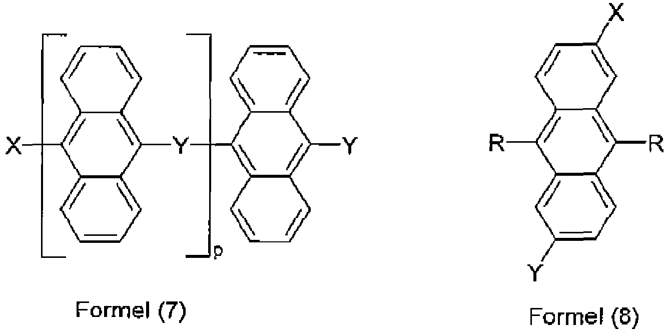 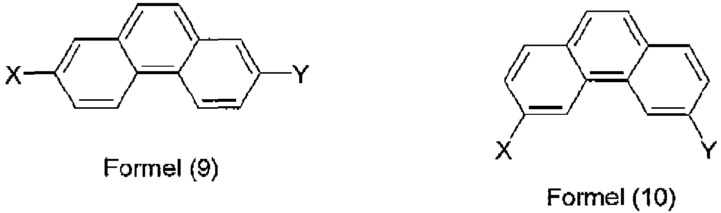 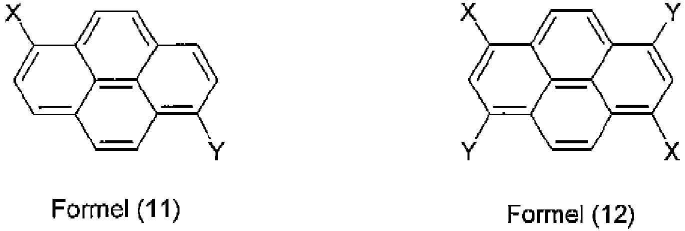 wobei X und Y dieselbe Bedeutung haben, wie in Anspruch 1 beschrieben, und wobei die Anthracen- bzw. Phenanthren- bzw. Pyreneinheiten durch einen oder mehrere Reste R substituiert sein können.

3. Verbindungen gemäß Anspruch 1 oder 2, **dadurch gekennzeichnet, dass** Ar³, gleich oder verschieden bei jedem Auftreten, für ein aromatisches oder heteroaromatisches Ringsystem mit 5 bis 20 aromatischen Ringatomen steht, das durch R substituiert oder unsubstituiert sein kann.

4. Verbindungen gemäß einem oder mehreren der Ansprüche 1 bis 3, **dadurch gekennzeichnet, dass** Q für eine lineare, verzweigte oder cyclische Alkylenkette mit 2 bis 15 C-Atomen steht, die durch R¹ substituiert sein kann und in der auch ein oder mehrere nicht benachbarte C-Atome durch N-R¹, O oder S ersetzt sein können und ein oder mehrere H-Atome durch F oder CN ersetzt sein können.

5. Verbindungen gemäß einem oder mehreren der Ansprüche 1 bis 4, **dadurch gekennzeichnet, dass** Q einen 3- bis 8-Ring aufspannt.

6. Verbindungen gemäß einem oder mehreren der Ansprüche 1 bis 5, **dadurch gekennzeichnet, dass** die Strukturen gemäß den Formeln (15) bis (20) ausgewählt sind aus den Strukturen gemäß den Formeln (21) bis (24), 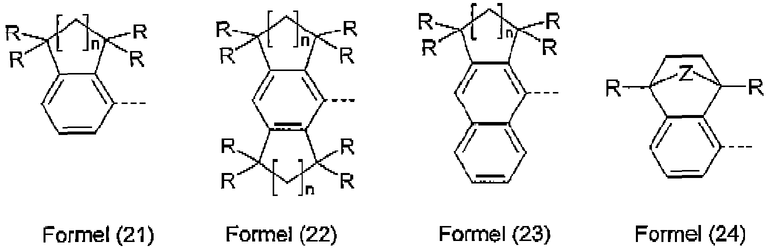 wobei R dieselbe Bedeutung hat, wie in Anspruch 1 beschrieben, und weiterhin gilt:
Z ist CR₂, O, S, NR, PR, P(=O)R, SiR₂ oder CR₂-CR₂;
n ist 1, 2 oder 3, bevorzugt 2;
dabei bedeutet die gestrichelte Bindung die Verknüpfung mit der Einheit Ar¹.

7. Verbindungen gemäß einem oder mehreren der Ansprüche 1 bis 6, **dadurch gekennzeichnet, dass** Q derart gewählt wird, dass es entweder keine benzylischen Protonen enthält oder dass ein Brückenkopf-C-Atom direkt mit Ar² verknüpft ist.

8. Konjugierte, teilkonjugierte oder nicht-konjugierte Polymere, Oligomere oder Dendrimere, enthaltend Wiederholeinheiten gemäß gemäß einem oder mehreren der Ansprüche 1 bis 7, wobei mindestens ein Rest R eine Bindung zum Polymer darstellt.

9. Polymere gemäß Anspruch 8, **dadurch gekennzeichnet, dass** die Polymere weitere Wiederholeinheiten enthalten, ausgewählt aus den Klassen der Fluorene, Spirobifluorene, Para-phenylene, Dihydrophenanthrene, Phenanthrene. Indenofluorene, Carbazole, Anthracene, Naphthaline, Triarylamine, Metallkomplexe oder Thiophene oder auch mehrere dieser Einheiten oder dass die Polymere Homopolymere aus Wiederholeinheiten gemäß einem oder mehreren der Ansprüche 1 bis 7 sind.

10. Mischungen enthaltend mindestens eine Verbindung gemäß einem oder mehreren der Ansprüche 1 bis 9 und einen oder mehrere Dotanden.

11. Mischungen gemäß Anspruch 10, **dadurch gekennzeichnet, dass** die Dotanden ausgewählt sind aus der Klasse der aromatischen Anthracenamine, der aromatischen Anthracendiamine, der aromatischen Pyrenamine, der aromatischen Pyrendiamine, der Monostyrylamine, der Distyrylamine, der Tristyrylamine, der Tetrastyrylamine, der Styrylphosphine, der Styrylether und der Arylamine.

12. Verwendung von Verbindungen oder Mischungen gemäß einem oder mehreren der Ansprüche 1 bis 11 in organischen elektronischen Vorrichtungen.

13. Organische elektronische Vorrichtungen, enthaltend Anode, Kathode und mindestens eine organische Schicht, welche mindestens eine Verbindung oder Mischung gemäß einem oder mehreren der Ansprüche 1 bis 11 enthält.

14. Organische elektronische Vorrichtungen gemäß Anspruch 13, ausgewählt aus der Gruppe bestehend aus organischen und polymeren Leuchtdioden (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren und organischen Laserdioden (O-Laser).

15. Organische Elektrolumineszenzvorrichtung gemäß Anspruch 13 oder 14, **dadurch gekennzeichnet, dass** außer der emittierenden Schicht noch eine oder mehrere weitere Schichten vorhanden sind, ausgewählt aus Lochinjektionsschicht, Lochtransportschicht, Ladungsblockierschicht, Elektronentransportschicht und/oder Elektroneninjektionsschicht.

16. Organische Elektrolumineszenzvorrichtungen gemäß einem oder mehreren der Ansprüche 13 bis 15, **dadurch gekennzeichnet, dass** die Verbindungen gemäß einem oder mehreren der Ansprüche 1 bis 9 als Host-Material für Dotanden, die aus dem Singulettzustand oder aus einem Zustand höherer Spinmultiplizität Licht emittieren, als Dotand, als Lochtransportmaterial, als Elektronentransportmaterial oder als Lochblockiermaterial verwendet werden.

## Claims

1. Compounds of the formula (1), 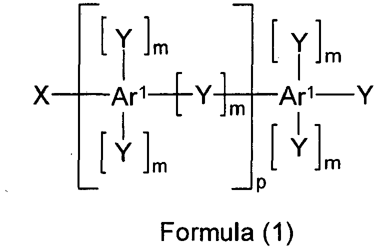 where the following applies to the symbols and indices used:
Ar¹ is on each occurrence, identically or differently, a condensed aryl or heteroaryl group selected from the group consisting of anthracene, phenanthrene, pyrene, naphthacene, chrysene, pentacene and perylene, which may be substituted by R;
X is on each occurrence, identically or differently, a group of one of the formulae (15) to (20) 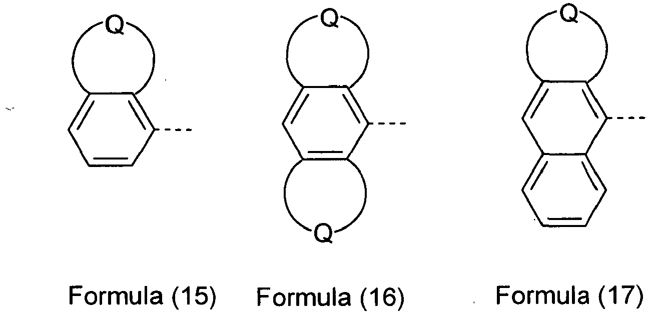 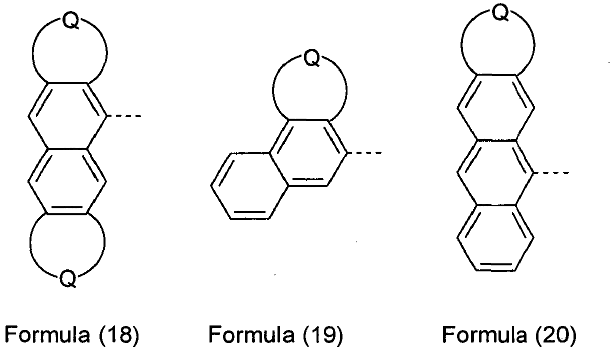 where the phenyl or naphthyl or anthryl unit may in each case also be substituted by R; the dashed bond here denotes the link to the unit Ar¹;
Y is on each occurrence, identically or differently, X, a group Ar³ or a group N(Ar³)₂, where the two radicals Ar³ may also be connected to one another by a single bond or a group O, S, N(R) or C(R)₂;
Ar³ is on each occurrence, identically or differently, an aromatic or heteroaromatic ring system, which may be substituted by one or more radicals R;
Q is on each occurrence, identically or differently, a linear, branched or cyclic alkylene or alkylidene group; Q here contains 1 to 20 C atoms and may be substituted by R¹, and one or more non-adjacent C atoms may also be replaced by N-R¹, O, S, O-CO-O, CO-O, -CR¹=CR¹- or -C≡C-, and one or more H atoms may be replaced by F, Cl, Br, I or CN;
R is on each occurrence, identically or differently, H, F, Cl, Br, I, CN, a straight-chain alkyl or alkoxy chain having 1 to 40 C atoms or a branched or cyclic alkyl or alkoxy group having 3 to 40 C atoms, which may in each case be substituted by R¹, in which, in addition, one or more non-adjacent C atoms may be replaced by N-R¹, O, S, O-CO-O, CO-O or -C≡C- and in which, in addition, one or more H atoms may be replaced by F, Cl, Br, I or CN, or an aromatic or heteroaromatic ring system having 5 to 40 aromatic ring atoms, which may also be substituted by one or more radicals R¹, or a combination of two, three or four of these systems; two or more radicals R here may also form a further mono- or polycyclic, aliphatic or aromatic ring system with one another;
R¹ is on each occurrence, identically or differently, H or a hydrocarbon radical having 1 to 20 C atoms, which may be aliphatic or aromatic or a combination of aliphatic and aromatic and in which, in addition, one or more H atoms may be replaced by F;
m is on each occurrence 0 or 1;
p is on each occurrence 0 or 1.

2. Structures of the formulae (7) to (12) according to Claim 1, 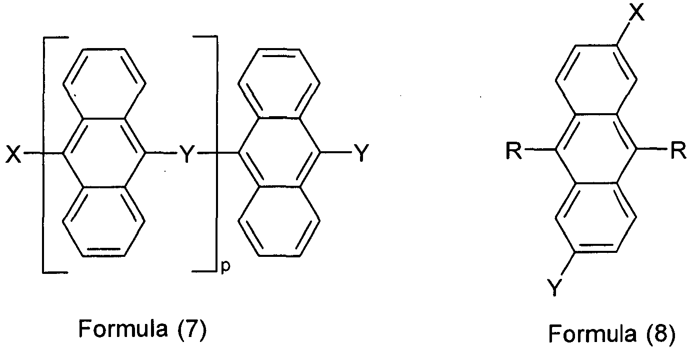 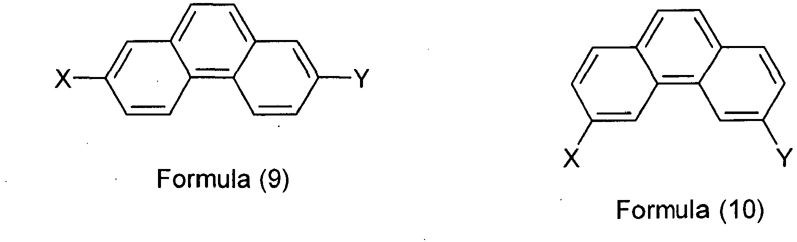 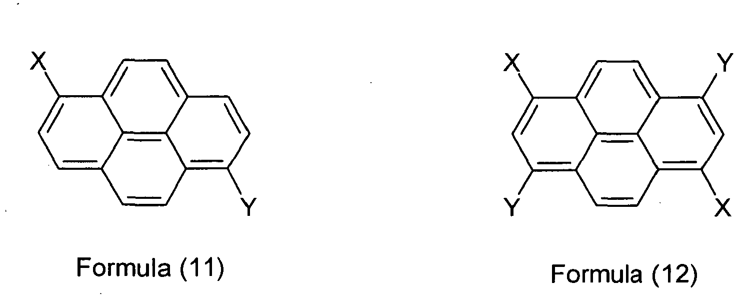 where X and Y have the same meaning as described in Claim 1, and where the anthracene or phenanthrene or pyrene units may be substituted by one or more radicals R.

3. Compounds according to Claim 1 or 2, **characterised in that** Ar³, identically or differently on each occurrence, stands for an aromatic or heteroaromatic ring system having 5 to 20 aromatic ring atoms, which may be substituted by R or unsubstituted.

4. Compounds according to one or more of Claims 1 to 3, **characterised in that** Q stands for a linear, branched or cyclic alkylene chain having 2 to 15 C atoms, which may be substituted by R¹ and in which, in addition, one or more non-adjacent C atoms may be replaced by N-R¹, O or S and one or more H atoms may be replaced by F or CN.

5. Compounds according to one or more of Claims 1 to 4, **characterised in that** Q forms a 3- to 8-membered ring.

6. Compounds according to one or more of Claims 1 to 5, **characterised in that** the structures of the formulae (15) to (20) are selected from the structures of the formulae (21) to (24), 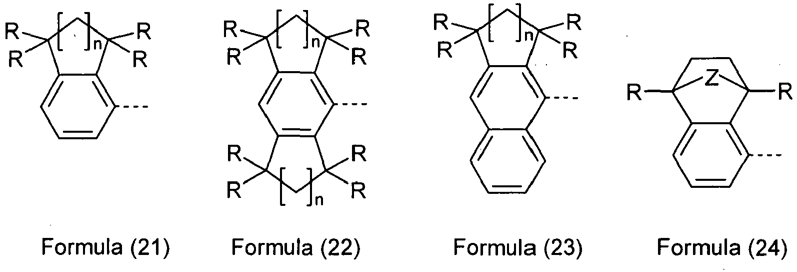 where R has the same meaning as described in Claim 1, and furthermore:
Z is CR₂, O, S, NR, PR, P(=O)R, SiR₂ or CR₂-CR₂;
n is 1, 2 or 3, preferably 2;
the dashed bond here denotes the link to the unit Ar¹.

7. Compounds according to one or more of Claims 1 to 6, **characterised in that** Q is selected in such a way that it either contains no benzylic protons or that a bridgehead C atom is linked directly to Ar².

8. Conjugated, partly conjugated or non-conjugated polymers, oligomers or dendrimers containing recurring units according to one or more of Claims 1 to 7, where at least one radical R represents a bond to the polymer.

9. Polymers according to Claim 8, **characterised in that** the polymers contain further recurring units selected from the classes of the fluorenes, spirobifluorenes, para-phenylenes, dihydrophenanthrenes, phenanthrenes, indenofluorenes, carbazoles, anthracenes, naphthalenes, triarylamines, metal complexes or thiophenes or also a plurality of these units or **in that** the polymers are homopolymers comprising recurring units according to one or more of Claims 1 to 7.

10. Mixtures comprising at least one compound according to one or more of Claims 1 to 9 and one or more dopants.

11. Mixtures according to Claim 10, **characterised in that** the dopants are selected from the class of the aromatic anthracenamines, the aromatic anthracenediamines, the aromatic pyrenamines, the aromatic pyrenediamines, the monostyrylamines, the distyrylamines, the tristyrylamines, the tetrastyrylamines, the styrylphosphines, the styryl ethers and the arylamines.

12. Use of compounds or mixtures according to one or more of Claims 1 to 11 in organic electronic devices.

13. Organic electronic devices comprising anode, cathode and at least one organic layer which comprises at least one compound or mixture according to one or more of Claims 1 to 11.

14. Organic electronic devices according to Claim 13, selected from the group consisting of organic and polymeric light-emitting diodes (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic photoreceptors and organic laser diodes (O-lasers).

15. Organic electroluminescent device according to Claim 13 or 14, **characterised in that**, apart from the emitting layer, one or more further layers selected from hole-injection layer, hole-transport layer, charge-blocking layer, electron-transport layer and/or electron-injection layer are also present.

16. Organic electroluminescent devices according to one or more of Claims 13 to 15, **characterised in that** the compounds according to one or more of Claims 1 to 9 are used as host material for dopants which emit light from the singlet state or from a state of higher spin multiplicity, as dopant, as hole-transport material, as electron-transport material or as hole-blocking material.

## Revendications

1. Composés de la formule (1) : 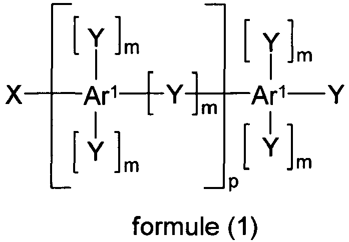 dans laquelle ce qui suit s'applique aux symboles et indices utilisés :
Ar¹ est, pour chaque occurrence, de manière identique ou différente, un groupe aryle ou hétéroaryle condensé qui est sélectionné parmi le groupe qui est constitué par anthracène, phénanthrène, pyrène, naphtacène, chrysène, pentacène et pérylène, lequel peut être substitué par R ;
X est, pour chaque occurrence, de manière identique ou différente, un groupe de l'une des formules (15) à (20) : 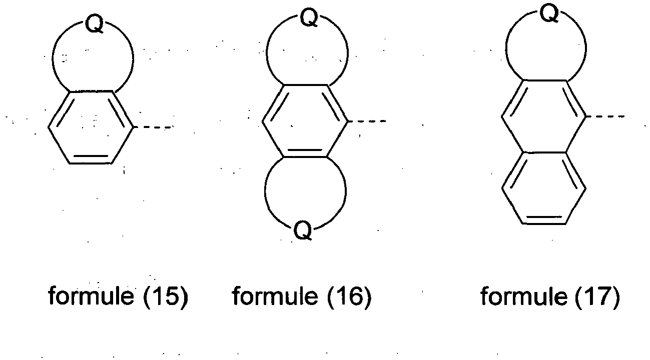 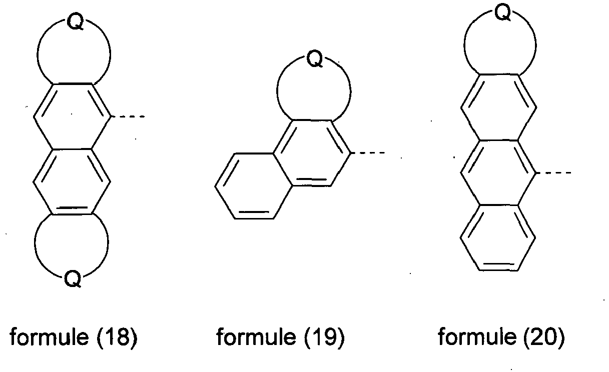 dans lesquelles l'unité phényle ou naphtyle ou anthryle peut, dans chaque cas, également être substituée par R ; le lien en pointillés ici représente la liaison sur l'unité Ar¹ ;
Y est, pour chaque occurrence, de manière identique ou différente, X, un groupe Ar³ ou un groupe N(Ar³)₂, où les deux radicaux Ar³ peuvent également être connectés l'un à l'autre par une liaison simple ou un groupe O, S, N(R) ou C(R)₂ ;
Ar³ est, pour chaque occurrence, de manière identique ou différente, un système de cycle aromatique ou hétéroaromatique, lequel peut être substitué par un radical ou plusieurs radicaux R ;
Q est, pour chaque occurrence, de manière identique ou différente, un groupe alkylène ou alkylidène linéaire, ramifié ou cyclique ; Q ici contient 1 à 20 atome(s) de C et peut être substitué par R¹, et un ou plusieurs atome(s) de C non adjacents peut/peuvent également être remplacé(s) par N-R¹, O, S, O-CO-O, CO-O, -CR¹=CR¹- ou -C≡C-, et un ou plusieurs atome(s) de H peut/peuvent être remplacé(s) par F, Cl, Br, I ouCN;
R est, pour chaque occurrence, de manière identique ou différente, H, F, Cl, Br, I, CN, une chaîne alkyle ou alcoxy en chaîne droite qui comporte 1 à 40 atome(s) de C ou un groupe alkyle ou alcoxy ramifié ou cyclique qui comporte 3 à 40 atomes de C, lequel peut, dans chaque cas, être substitué par R¹, où, en outre, un ou plusieurs atome(s) de C non adjacents peut/peuvent être remplacé(s) par N-R¹, O, S, O-CO-O, CO-O ou -C≡C- et où, en outre, un ou plusieurs atome(s) de H peut/peuvent être remplacé(s) par F, Cl, Br, I ou CN, ou un système de cycle aromatique ou hétéroaromatique qui comporte 5 à 40 atomes de cycle aromatique, lequel peut également être substitué par un radical ou plusieurs radicaux R¹, ou une combinaison de deux, trois ou quatre de ces systèmes ; deux radicaux R ou plus ici peuvent également former un autre système de cycle aliphatique ou aromatique monocyclique ou polycyclique l'un avec l'autre ou les uns avec les autres ;
R¹ est, pour chaque occurrence, de manière identique ou différente, H ou un radical hydrocarbone qui comporte 1 à 20 atome(s) de C, lequel peut être aliphatique ou aromatique ou une combinaison d'aliphatique et d'aromatique et où, en outre, un ou plusieurs atome(s) de H peut/peuvent être remplacé(s) par F ;
m est, pour chaque occurrence, 0 ou 1 ;
p est, pour chaque occurrence, 0 ou 1.

2. Structures des formules (7) à (12) selon la revendication 1 : 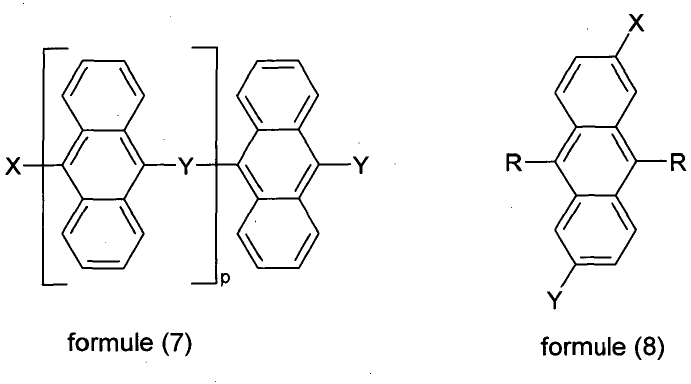 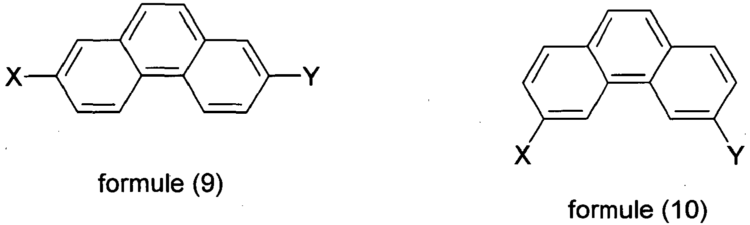 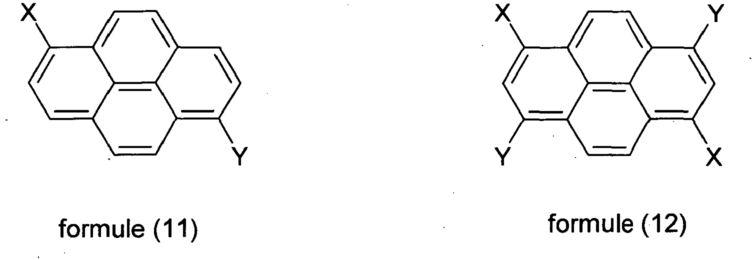 dans lesquelles X et Y présentent la même signification que décrit selon la revendication 1, et où les unités anthracène ou phénanthrène ou pyrène peuvent être substituées par un radical ou plusieurs radicaux R.

3. Composés selon la revendication 1 ou 2, **caractérisés en ce que** Ar³, de manière identique ou différente pour chaque occurrence, représente un système de cycle aromatique ou hétéroaromatique qui comporte 5 à 20 atomes de cycle aromatique, lequel peut être substitué par R ou non substitué.

4. Composés selon une ou plusieurs des revendications 1 à 3, **caractérisés en ce que** Q représente une chaîne alkylène linéaire, ramifiée ou cyclique qui comporte 2 à 15 atomes de C, laquelle peut être substituée par R¹ et où, en outre, un ou plusieurs atome(s) de C non adjacents peut/peuvent être remplacé(s) par N-R¹, par O ou par S et un ou plusieurs atome(s) de H peut/peuvent être remplacé(s) par F ou par CN.

5. Composés selon une ou plusieurs des revendications 1 à 4, **caractérisés en ce que** Q forme un cycle à 3 à 8 éléments.

6. Composés selon une ou plusieurs des revendications 1 à 5, **caractérisés en ce que** les structures des formules (15) à (20) sont sélectionnées parmi les structures des formules (21) à (24) : 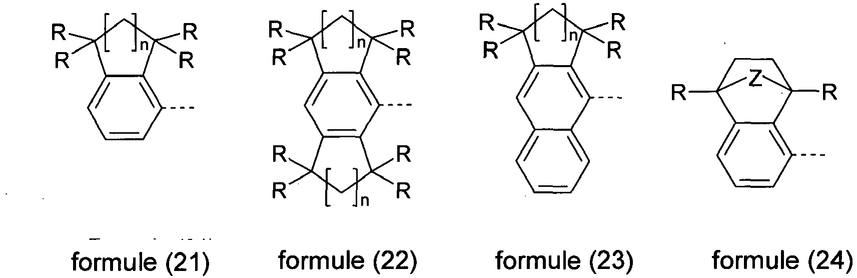 dans lesquelles R présente la même signification que décrit selon la revendication 1, et en outre :
Z est CR₂, O, S, NR, PR, P(=O)R, SiR₂ ou CR₂-CR₂ ;
n est 1, 2 ou 3, de façon préférable 2 ;
le lien en pointillés ici représente la liaison sur l'unité Ar¹.

7. Composés selon une ou plusieurs des revendications 1 à 6, **caractérisés en ce que** Q est sélectionné de sorte que soit il ne contient pas de protons benzyliques, soit un atome de C en tête de pont est lié directement à Ar².

8. Polymères, oligomères ou dendrimères conjugués, partiellement conjugués ou non conjugués contenant des unités récurrentes selon une ou plusieurs des revendications 1 à 7, où au moins un radical R représente une liaison sur le polymère.

9. Polymères selon la revendication 8, **caractérisés en ce que** les polymères contiennent d'autres unités récurrentes qui sont sélectionnées parmi les classes des fluorènes, des spirobifluorènes, des paraphénylènes, des dihydrophénanthrènes, des phénanthrènes, des indénofluorènes, des carbazoles, des anthracènes, des naphtalènes, des triarylamines, des complexes métalliques ou des thiophènes ou également une pluralité de ces unités ou **en ce que** les polymères sont des homopolymères qui comprennent des unités récurrentes selon une ou plusieurs des revendications 1 à 7.

10. Mélanges comprenant au moins un composé selon une ou plusieurs des revendications 1 à 9 et un ou plusieurs dopant(s).

11. Mélanges selon la revendication 10, **caractérisés en ce que** les dopants sont sélectionnés parmi la classe des anthracènamines aromatiques, des anthracènediamines aromatiques, des pyrènamines aromatiques, des pyrènediamines aromatiques, des monostyrylamines, des distyrylamines, des tristyrylamines, des tétrastyrylamines, des styrylphosphines, des styryléthers et des arylamines.

12. Utilisation de composés ou de mélanges selon une ou plusieurs des revendications 1 à 11 dans des dispositifs électroniques organiques.

13. Dispositifs électroniques organiques comprenant une anode, une cathode et au moins une couche organique qui comprend au moins un composé ou un mélange selon une ou plusieurs des revendications 1 à 11.

14. Dispositifs électroniques organiques selon la revendication 13, sélectionnés parmi le groupe qui est constitué par des diodes émettrices de lumière organiques et polymériques (OLED, PLED), des transistors à effet de champ organiques (O-FET), des transistors à film mince organiques (O-TFT), des transistors à émission de lumière organiques(O-LET), des circuits intégrés organiques (O-IC), des cellules solaires organiques (O-SC), des dispositifs à extinction de champ organiques (O-FQD), des cellules électrochimiques à émission de lumière (LEC), des photorécepteurs organiques et des diodes laser organiques (O-laser).

15. Dispositif électroluminescent organique selon la revendication 13 ou 14, **caractérisé en ce que**, indépendamment de la couche d'émission, une ou plusieurs autre(s) couche(s) qui est/sont sélectionnée(s) parmi une couche d'injection de trous, une couche de transport de trous, une couche de blocage de charges, une couche de transport d'électrons et/ou une couche d'injection d'électrons est/sont également présente(s).

16. Dispositifs électroluminescents organiques selon une ou plusieurs des revendications 13 à 15, **caractérisés en ce que** les composés selon une ou plusieurs des revendications 1 à 9 sont utilisés en tant que matériau hôte pour des dopants qui émettent de la lumière depuis l'état singulet ou depuis un état de multiplicité de spin plus élevée, en tant que dopant, en tant que matériau de transport de trous, en tant que matériau de transport d'électrons ou en tant que matériau de blocage de trous.
